# EUROPÄISCHE PATENTSCHRIFT

(11) **EP 4 204 033 B1**
(45) Veröffentlichungstag und Bekanntmachung des Hinweises auf die Patenterteilung: **21.10.2026**
(21) Anmeldenummer: 21769669.9
(22) Anmeldetag: 23.08.2021
(51) Int. Cl.: A61M 1/36, B65D 1/22, A61B 50/00

(54) **VERPACKUNGSSYSTEM FÜR MEDIZINISCHES SCHLAUCHSET**
PACKING SYSTEM FOR MEDICAL FLEXIBLE TUBING SET
SYSTÈME D'EMBALLAGE DE TUYAUX FLEXIBLES POUR APPLICATION MÉDICALE

(30) Priorität: 25.08.2020 DE 102020005197
(43) Veröffentlichungstag der Anmeldung: 05.07.2023
(73) Patentinhaber: Xenios AG, 74076 Heilbronn (DE)
(72) Erfinder: SCHON, Hans-Günther, 67577 Alsheim (DE); WATMANN, Alice, 71726 Benningen (DE); GÜNTHER, Detlef, 70794 Filderstadt (DE)
(74) Vertreter: Graf von Stosch Patentanwaltsgesellschaft mbH
(86) Internationale Anmeldenummer: PCT/EP2021/073258
(87) Internationale Veröffentlichungsnummer: WO 2022/043255

(56) Entgegenhaltungen:
- WO-A1-2019/246307
- CN-A- 110 697 195
- US-B2- 8 672 172

## Beschreibung

Die vorliegende Erfindung betrifft Verpackungssysteme für medizinische Schlauchsets, insbesondere für extrakorporale Therapien. Die Erfindung betrifft auch die Verwendung eines solchen Verpackungssystems zum Verpacken eines medizinischen Schlauchsets sowie ein Verfahren zum Verpacken eines medizinischen Schlauchsets.

Medizinische Schlauchsets werden bei der Versorgung von Patienten vielseitig eingesetzt. So können medizinische Schläuche der Leitung von Flüssigkeiten (wie Blut, Kochsalzlösung und/oder Medikamente) oder Gasen (bspw. Sauerstoff) dienen. Medizinische Schlauchsets umfassen in der Regel zusätzlich zu den Schläuchen weitere Artikel, die zusammen mit den medizinischen Schläuchen verwendet werden. Daher werden medizinische Schlauchsets in der Regel anwendungsspezifisch konfektioniert. So weisen bspw. Schlauchsets zur extrakorporalen Blutbehandlung (bspw. zur Entfernung von Kohlendioxid aus dem Blut bzw. zur Versorgung des Bluts mit Sauerstoff mit Oxygenatoren) in der Regel Schläuche zur Blutführung, aber auch gasführende Schläuche (bspw. zum Sauerstofftransport) auf. Häufig werden medizinische Schlauchsets mit den für die Anwendung vorgesehenen Geräten (im obigen Beispiel z.B. einen Oxygenator) ausgeliefert.

Für die Anwendung im medizinischen Bereich ist die Sterilität der Medizinprodukte von großer Bedeutung. So muss sichergestellt werden, dass die Sterilität eines Medizinprodukts, wie bspw. eines medizinischen Schlauchsets, von der Herstellung bis zur Verwendung im Rahmen einer Behandlung, bspw. der extrakorporalen Therapie eines Patienten, gewährleistet ist. Daher sind die Anforderungen an die Verpackung von Medizinprodukten besonders hoch, da eine Sterilbarriere gegenüber der Umgebung sichergestellt werden muss, die während realistischer Lager- und Transportbedingungen aufrecht erhalten bleibt. Darüber hinaus muss die Verpackung gewährleisten, dass das Medizinprodukt während des Transports nicht beschädigt wird.

Bei herkömmlichen Verpackungen für Medizinprodukte müssen die Medizinprodukte nach dem Auspacken in der Regel einzeln an den Ort der Verwendung, bspw. in den Therapiebereich wie eine Intensivstation, verbracht werden. Dabei werden die Medizinprodukte durch Berührung der Gefahr einer Kontamination ausgesetzt. Darüber hinaus besteht die Gefahr der Beschädigung einzelner Produktbestandteile durch ungeschützte, herunterhängende Baugruppen beim Transport in der kontrollierten Zone (OP). Zudem wird bei der Verpackung von Sets häufig Klettband eingesetzt, mit dem die Fixierung des Produkts nicht standardisiert, d.h. mittels definierter reproduzierbarer Anzugsmomente, erfolgen kann, so dass Transportschäden auftreten können und lange Rüstzeiten für die Konfektionierung nötig sind. Darüber hinaus erfordern herkömmliche Verpackungen für Medizinprodukte häufig eine umständliche und wenig gebrauchstaugliche Entnahme der Produkte aus der Verpackung.

WO 2019/246307 A1 und CN 110 697 195 A beschreiben ein Verpackungssystem für ein medizinisches Schlauchset mit einer Wanne zur Aufnahme und reversiblen Befestigung einer Einlage.

Vor diesem Hintergrund ist es Aufgabe der vorliegenden Erfindung, die Nachteile des Stands der Technik zu überwinden und ein verbessertes Verpackungssystem für ein medizinisches Schlauchset bereitzustellen. Insbesondere kann hierbei durch geeignete Lagerung die Sicherheit und Funktionalität der verpackten Produkte während des Transports gewährleistet werden. Darüber hinaus kann die Sterilität der medizinischen Produkte, insbesondere von medizinischen Schläuchen, von der Verpackung bis zu deren medizinischer Anwendung erhalten werden. Zudem wird durch den modularen Aufbau des Verpackungssystems eine definiert reproduzierbare und nutzerfreundliche Entnahme der medizinischen Produkte im Wesentlichen in der Reihenfolge ihrer Anwendung ermöglicht. Der modulare Aufbau ermöglicht außerdem eine einfache Anpassung des Verpackungssystems an verschiedene medizinische Schlauchsets, bspw. mit verschiedenen oder unterschiedlich dimensionierten Komponenten, wobei jeweils lediglich ein (oder wenige) Modul(e) des Verpackungssystems angepasst werden müssen.

Die Aufgabe wird durch die unabhängigen Ansprüche gelöst. Vorteilhafte Weiterbildungen ergeben sich aus den Unteransprüchen, der Beschreibung und den Figuren. Bevorzugte Ausführungsformen des erfindungsgemäßen Verpackungssystems entsprechen bevorzugten Ausführungsformen der erfindungsgemäßen Verwendung, des erfindungsgemäßen Verfahrens bzw. des erfindungsgemäßen Befestigungsmittels und umgekehrt.

Im Folgenden bedeuten Begriffe wie "ca.", "ungefähr", "etwa" oder "im Wesentlichen" in Bezug auf numerische Angaben in der Regel ± 10% (bevorzugt ± 7%, mehr bevorzugt ± 5%, noch mehr bevorzugt ± 2%, besonders bevorzugt ± 1%), es sei denn diese Begriffe sind für einen speziellen Fall anders definiert.

Fachausdrücke haben in der Regel ihre gewöhnliche, fachliche Bedeutung, es sei denn sie werden hierin anders definiert.

Die vorliegende Erfindung stellt ein Verpackungssystem für ein medizinisches Schlauchset zur extrakorporalen Therapie bereit, wobei das Verpackungssystem die folgenden Komponenten umfasst:
- eine Wanne, die zur Aufnahme einer Einlage sowie zur reversiblen Befestigung der Einlage in der Wanne ausgebildet ist, und
- eine Einlage mit einer oder mehreren Vertiefungen zur Aufnahme (von Komponenten) des Schlauchsets,
   wobei zwischen Wanne und Einlage ein Hohlraum ausgebildet ist, und
- ein Befestigungsmittel zur reversiblen Befestigung der Einlage in der Wanne.

Das Verpackungssystem nach der Erfindung ist durch die Ansprüche 1 bis 15 definiert.

Das erfindungsgemäße Verpackungssystem ist insbesondere modular aufgebaut, so dass eine einfache Anpassung des Verpackungssystems an verschiedene medizinische Schlauchsets, bspw. mit verschiedenen oder unterschiedlich dimensionierten Komponenten ermöglicht wird, wobei in der Regel jeweils lediglich ein (oder wenige) Modul(e) des Verpackungssystems angepasst werden müssen. So können bei einem derartigen Verpackungssystem Wannen vorteilhaft dieselbe Größe aufweisen, auch wenn sie zur Verpackung verschiedener Schlauchsets (unterschiedlicher Größe oder unterschiedlichen Umfangs) verwendet werden. Dabei kann bspw. die Einlage für die verschiedenen Schlauchsets unterschiedlich gestaltet sein. So ermöglicht das erfindungsgemäße Verpackungssystem eine große Flexibilität bezüglich der Verpackung verschiedener Schlauchsets, da jeweils (nur) eine einzige oder wenige Komponente(n) des Verpackungssystems (bspw. die Einlage - aber bspw. nicht die anderen Komponenten des Verpackungssystems) an die jeweils unterschiedlichen Schlauchsets angepasst werden muss.

Zudem wird durch den modularen Aufbau des Verpackungssystems eine definiert reproduzierbare und nutzerfreundliche Entnahme des Schlauchsets im Wesentlichen in der Reihenfolge ihrer Anwendung ermöglicht.

Insbesondere kann das Schlauchset vollständig auf der Einlage verpackt werden. Da die Einlage reversibel in der Wanne befestigt werden kann, ist ein einfaches Verpacken (und Auspacken) des Schlauchsets auf der Einlage außerhalb der Wanne möglich. Das auf der Einlage verpackte Schlauchset kann dann in einem weiteren Schritt (mit der Einlage) in die Wanne eingebracht und dort befestigt werden. Ebenso kann beim Auspacken zunächst die reversible Befestigung der Einlage gelöst werden, so dass die Einlage mit dem (verpackten) Schlauchset aus der Wanne entnommen werden kann, und danach das Schlauchset (außerhalb der Wanne) von der Einlage entpackt werden kann. Dies ermöglicht einen einfachen Transport des (auf der Einlage verpackten) Schlauchsets in sterile Bereiche (Anwendungsbereiche), in denen eine ggf. äußerlich kontaminierte Wanne unerwünscht ist. Die verschiedenen Komponenten des Schlauchsets müssen dann nicht umständlich einzeln in besonders sterile Bereiche verbracht werden, sondern können mitsamt der (sterilen) Einlage einfach dorthin transportiert werden.

Ein beispielhaftes medizinisches Schlauchset, das im erfindungsgemäßen Verpackungssystem verpackt werden kann, kann einen Schlauch zur Blutführung, einen Schlauch zur Gasführung, und ggf. einen Oxygenator und/oder einen Pumpenkopf umfassen. Weiterhin können verschiedene zusätzliche Artikel, die in der Regel zusammen mit dem medizinischen Schlauchset verwendet werden, wie bspw. Schlauchklemmen, eine Spritze, Kabelbinder etc., ebenfalls im erfindungsgemäßen Verpackungssystem verpackt werden (und daher im Lieferumfang enthalten sein).

Der Hohlraum, der zwischen Wanne und Einlage (insbesondere in befestigtem Zustand der Einlage in der Wanne) ausgebildet ist, dient einem sicheren Transport empfindlicher Komponenten des Schlauchsets (bspw. eines Oxygenators und/oder eines Pumpenkopfes), da durch den Hohlraum zwischen Einlage und Wanne Vibrationen abgefedert werden können, um das Schlauchset zu schützen.

Da das Verpackungssystem der Verpackung eines medizinischen Schlauchsets dient, wie es im medizinischen Bereich, bspw. auf einer Intensivstation, verwendet wird, ist das Verpackungssystem (sowie das darin verpackte Schlauchset) vorzugsweise steril, insbesondere innerhalb der Wanne. Vorzugsweise werden die Komponenten des Verpackungssystems (und des Schlauchsets) vor und/oder während des Verpackens des Schlauchsets im Verpackungssystem sterilisiert. Danach wird die Wanne (mit dem darin verpackten Schlauchset) verschlossen, vorzugsweise versiegelt, bspw. mittels einer Folie. Das Material der Versiegelung (Folie) ist vorzugsweise sowohl zur Versiegelung als auch zur Sterilisation geeignet, bspw. Tyvek^{®} (PE-HD). Die Sterilität des Verpackungssystems innerhalb der Wanne (und des darin verpackten Schlauchsets) bleibt so auch bei äußerlichen Kontaminationen erhalten (bspw. während Transport und/oder Lagerung), auch wenn die Wanne äußerlich ihre Sterilität verlieren kann.

Die Einlage des Verpackungssystems weist eine oder mehrere Vertiefungen zur Aufnahme (von Komponenten) des Schlauchsets auf. Bspw. kann die Einlage Vertiefungen zur Aufnahme eines oder mehrerer Schläuche aufweisen. Darüber hinaus kann die Einlage einen oder mehrere Aufnahmeabschnitt(e) aufweisen, bspw. zur Aufnahme für einen Oxygenator (und/oder einen Pumpenkopf), aufweisen. Im Gegensatz zu den Vertiefungen können Aufnahmeabschnitte sowohl Vertiefungen (in der Einlage) als auch Erhebungen (aus der Einlage) umfassen.

Die Form der Vertiefung(en) und des/der Aufnahmeabschnitt(e) kann vorteilhaft an die Form des darin/darauf aufzunehmenden Gegenstands (Komponente des Schlauchsets) angepasst sein. Bspw. kann die Form der Vertiefung oder des Aufnahmeabschnitts (einem Teilabschnitt) der äußeren Form des aufzunehmenden Gegenstands entsprechen. So können Gegenstände (Schlauchset-Komponenten) in/an/auf der Einlage durch die Vertiefung(en) bzw. Aufnahmeabschnitt(e) bspw. im Wesentlichen formschlüssig aufgenommen/angeordnet werden.

Die Form der Vertiefung(en) muss jedoch nicht der äußeren Form des aufzunehmenden Gegenstands entsprechen. So kann die Einlage eine oder mehrere Vertiefung(en) aufweisen, die nicht der äußeren Form des aufzunehmenden Gegenstands entsprechen, sondern die eine gewisse Flexibilität in der Aufnahme des Gegenstands ermöglichen. Dies kann bspw. bei der Aufnahme von Schläuchen vorteilhaft sein, um eine leichte Ver- und Entpackung zu ermöglichen. Insbesondere müssen bspw. Schläuche zum sicheren Transport in der Regel nicht über ihre gesamte Länge (an jeder Stelle) fixiert sein. So kann das Verpackungssystem eine punktuelle Fixierung von Gegenständen (bei Schläuchen bspw. durch ein Schott) ermöglichen. Bspw. können für Schläuche vorgesehenen Vertiefungen vorteilhaft größer als der Schlauchumfang ausgelegt sein, um einen gewissen Spielraum beim Ver- bzw. Auspacken des Schlauches zu ermöglichen und ein Abknicken des Schlauchs zu verhindern. So können zur Aufnahme von Schläuchen vorgesehene Vertiefungen bspw. so ausgelegt sein, dass mehrere Lagen oder Schlaufen des Schlauchs bzw. mehrere Schläuche darin aufgenommen werden können. Vorteilhaft sind bspw. im Wesentlichen runde oder elliptische Vertiefungen bzw. Aufnahmen für Schläuche. In einer für einen Schlauch vorgesehenen Vertiefung oder Aufnahmeabschnitt kann in etwa zentral eine Erhebung angeordnet sein, um die der Schlauch gewickelt werden kann.

In der Einlage können mehrere Vertiefungen separat (voneinander getrennt) oder miteinander verbunden (bspw. durch kanalartige Verbindungsvertiefungen) vorliegen. Während separat vorliegende Vertiefungen in der Regel eine vollständig umlaufende Innenwand (ohne Aussparung oder Unterbrechung) aufweisen, weisen miteinander verbundene Vertiefungen seitliche Aussparungen auf, über die sie - bspw. mittels kanalartiger Verbindungsvertiefung - miteinander in Verbindung stehen. So kann bspw. ein und derselbe Schlauch in mehreren Vertiefungen verpackt werden, indem eine oder mehrere Schlaufen des Schlauchs in einer ersten Vertiefung verpackt werden, der Schlauch dann durch eine kanalartige Verbindungsvertiefung einer zweiten Vertiefung geführt wird und in dieser (zweiten) Vertiefung wiederum eine oder mehrere Schlaufen des Schlauchs verpackt werden.

Die Wanne weist in der Regel einen Boden und eine Wand auf, wobei hierin die Innenseite der Wand auch als "Innenwand" und die Außenseite der Wand auch als "Außenwand" bezeichnet wird. Die Wand wird hierin auch als "Seitenwand" bzw. "Seitenwände" bezeichnet.

Die Größe der Wanne kann durch die Größe der Einlage bzw. durch die Größe des verpackten Schlauchsets bestimmt werden. Eine beispielhafte Wanne zur Verpackung eines medizinischen Schlauchsets mit einem Schlauch zur Blutführung, einem Schlauch zur Gasführung, einem Oxygenator, sowie einem Pumpenkopf kann eine Länge von etwa 50 - 65 cm, bevorzugt 55 - 60 cm, bspw. 56 - 57 cm; eine Breite von etwa 40 - 55, bevorzugt 45 - 50 cm, bspw. etwa 47 cm; und/oder eine Höhe von etwa 12 - 20 cm, bevorzugt 14 - 18 cm, bspw. etwa 16 - 16,5 cm, aufweisen. Die Wanddicke der Wanne kann etwa 2 - 3 mm (bspw. etwa 2,5 mm) betragen.

Wannen für verschiedene medizinische Schlauchsets können vorteilhaft dieselbe Größe aufweisen, wobei ggf. die Einlage für verschiedene Schlauchsets unterschiedlich gestaltet sein kann (jedoch so, dass die Einlage jeweils in die "universelle" Wanne passt). So ermöglicht das erfindungsgemäße Verpackungssystem eine große Flexibilität bezüglich der Verpackung verschiedener Schlauchsets, da jeweils (nur) die Einlage (aber bspw. nicht die anderen Komponenten des Verpackungssystems) an die jeweils unterschiedlichen Schlauchsets angepasst werden muss.

Die Form der Wanne wird in der Regel durch deren Grundfläche (Boden) vorgegeben und ist nicht eingeschränkt. Jedoch sind Wannen mit im Wesentlichen rechteckigen Grundflächen besonders vorteilhaft für Lagerung und Transport, da sie in platzsparender Weise transportiert und gelagert werden können. Eine "im Wesentlichen rechteckige" Grundfläche wird durch vier (im Wesentlichen) rechtwinklig zueinander angeordnete Seiten gebildet, wobei die jeweils gegenüberliegenden Seiten (im Wesentlichen) parallel zueinander angeordnet sind. Die Seiten können Ausnehmungen, Ausstülpungen, Biegungen oder ähnliches aufweisen, solange die jeweiligen (gedachten) Grundlinien der Seiten die o.g. Anforderungen erfüllen. Die "Ecken" des Rechtecks können abgerundet oder abgeschrägt sein.

Die Wanne kann bspw. aus einem Kunststoff, wie PET (Polyethylenterephthalat) oder PETG (mit Glykol modifiziertes PET), gefertigt sein. Die Wanne kann auf verschiedene Weise hergestellt werden, bspw. mittels Tiefziehverfahren oder 3D-Druck.

Die Wände der Wanne können im Wesentlichen senkrecht zum Boden der Wanne ausgebildet sein. "Im Wesentlichen senkrecht" schließt Abweichungen von der im Vergleich zum Boden senkrechten Achse von bis zu 20° ein; bevorzugt Abweichungen von der im Vergleich zum Boden senkrechten Achse bis zu 15°, mehr bevorzugt bis zu 10°, noch mehr bevorzugt bis zu 5°. So kann die Wanne vorteilhaft leicht nach außen geneigte (Seiten)Wände aufweisen. Bspw. können die Wände um bis zu 20° (bevorzugt bis zu 15°, mehr bevorzugt bis zu 10°, noch mehr bevorzugt bis zu 5°, besonders bevorzugt bis zu 4° oder 3°, bspw. bis zu 2° oder 1°) nach außen geneigt sein. Auf diese Weise weist die Wanne im Vergleich eine Grundfläche (am Boden der Wanne) auf, die kleiner ist als die durch den (im Wesentlichen horizontalen) oberen Rand der Wanne gebildete Ebene. Eine solche kleinere Grundfläche (am Boden) im Vergleich zu der durch den oberen Rand gebildeten Ebene kann auch durch Abstufungen in den Seitenwänden der Wanne erzielt werden. Abstufungen der Seitenwände der Wanne können sowohl in senkrechten Seitenwänden als auch vorteilhaft in leicht nach außen geneigten Seitenwänden vorhanden sein. Derartige Wannen, bei denen die Grundfläche (am Boden) kleiner ist als die durch den oberen Rand gebildete Ebene sind vorteilhaft, da sie leicht gestapelt und daher platzsparend gelagert werden können.

Der obere Rand der Wanne kann vorteilhaft im Wesentlichen horizontal ausgebildet sein. Vorzugsweise ist der obere Rand (im Vergleich zur Wanddicke der Wanne) in horizontaler Ebene verbreitert, bspw. indem die Seitenwände der Wanne horizontal nach außen "umgebogen" sind. So kann auf einfache Weise eine Abdeckung (bspw. eine Folie) zur Versiegelung der Wanne aufgebracht werden. So kann der obere Rand der Wanne eine Breite von ca. 5 bis 20 mm, bevorzugt 7 - 15 mm, mehr bevorzugt 10 - 12 mm, bspw. 10,5 - 11 mm, aufweisen.

Bevorzugt sind Wanne und/oder Einlage derart ausgelegt, dass alle Komponenten des Schlauchsets, sowie ggf. alle weiteren zu verpackenden Gegenstände, (vollständig) auf (d.h. oberhalb) der Einlage verpackt werden können. Auf diese Weise profitieren alle zu verpackenden Komponenten bzw. Gegenstände von dem Hohlraum (zur Abfederung) zwischen Wanne und Einlage. Darüber hinaus können alle verpackten Komponenten und Gegenstände auf diese Weise einfach mit der Einlage aus der Wanne entnommen (und ggf. weiter transportiert) werden.

Form (Umriss) und/oder Größe der Einlage können vorteilhaft mit der Form und/oder Größe (des Innenraums) der Wanne korrespondieren. Mit anderen Worten, die Einlage erstreckt sich vorteilhaft im Wesentlichen über den gesamten Bereich (bspw. Boden) der Wanne. Insbesondere kann die Wanne (im Inneren) eine Länge und eine Breite aufweisen, die die Länge und die Breite der Einlage jeweils um nicht mehr als 3 cm, bevorzugt nicht mehr als 2 cm, mehr bevorzugt nicht mehr als 1 cm, besonders bevorzugt nicht mehr als 0,5 cm übersteigt. Vorteilhaft ist die Einlage (in Länge und Breite) nur geringfügig kleiner als die Wanne. Dies ermöglicht, dass sich die Einlage vorteilhaft im Wesentlichen über den gesamten Bereich (bspw. Boden) der Wanne erstreckt (und damit eine platzsparende Verpackung ermöglicht), aber auch leicht in die Wanne hinein verbracht und aus ihr heraus genommen werden kann. Dabei ist es besonders vorteilhaft, wenn die Einlage auf eine im Wesentlichen horizontale Auflagefläche an der Innenwand der Wanne aufgelegt werden kann.

Die Wanne kann vorteilhaft an ihrer Innenwand eine im Wesentlichen horizontale Auflagefläche (zur Auflage der Einlage) aufweisen. Die Auflagefläche kann sich (über alle Innenwände der Wanne) im Wesentlichen umlaufend erstrecken. Alternativ kann die Auflagefläche an der Innenwand der Wanne bspw. durch jeweils einen oder mehrere (horizontale) Auflagepunkte an jeder der vier Innenwandseiten der Wanne realisiert werden. Die Auflagefläche kann bspw. durch eine Abstufung in der (Innen-)Wand erreicht werden. Eine solche Abstufung in der (Innen-)Wand der Wanne hat den Vorteil, dass die Wanne so (besser) stapelbar ist. Insbesondere wird durch die Abstufung ein Abstand gewahrt, der beim Entstapeln einer Vakuumbildung entgegenwirkt. Alternativ kann die Auflagefläche auch durch einen Vorsprung gebildet werden. Die Auflagefläche kann bspw. eine Breite von ca. 3 bis 25 mm aufweisen.

Auf dieser Auflagefläche kann die Einlage, insbesondere der Außenrand auf der Unterseite der Einlage, (im Wesentlichen formschlüssig) abgelegt werden, so dass ein horizontales Verrutschen der Einlage in der Wanne verhindert oder zumindest begrenzt wird. Die Auflagefläche ist vorteilhaft in der unteren Hälfte der Wandhöhe der Wanne, vorzugsweise im unteren Drittel der Wandhöhe, angeordnet. Vorzugsweise ist die Auflagefläche nicht (tiefer als) im unteren Fünftel, bevorzugt nicht (tiefer als) im unteren Viertel der Wandhöhe der Wanne angeordnet. Auf diese Weise verbleibt oberhalb der Auflagefläche (Einlage) ausreichend Platz zur Anordnung des Schlauchsets und unterhalb der Auflagefläche (unterhalb der Einlage) verbleibt ausreichend Spielraum zur Anordnung der Vertiefungen in der Einlage, ohne dass die (Unterseiten der) Vertiefungen den Wannenboden berühren. Beim Verpacken des Schlauchsets kann die Einlage auf die Auflagefläche der Innenwand der Wanne aufgelegt werden, so dass die Unterseite des Außenrands der Einlage zumindest teilweise mit der Auflagefläche der Wanne in Kontakt ist. Auf diese Weise kann die Einlage in der Wanne in vertikaler Richtung stabilisiert werden. Die Wanne ist dabei vorteilhaft so dimensioniert, dass die Einlage gleichzeitig auf Auflageflächen an allen Innenwandseiten (bspw. an allen vier Innenwandseiten bei rechteckiger Grundform) aufliegen kann und die Innenwände der Wanne eine horizontale Bewegung der Einlage limitieren bzw. im Wesentlichen verhindern. Auf diese Weise wird eine (unbeabsichtigte) horizontale Bewegung (wie ein Verrutschen) der Einlage in der Wanne (zumindest teilweise) verhindert oder begrenzt. In einigen Ausführungsformen berührt die Einlage nicht den Boden der Wanne.

Besonders vorteilhaft ist es, wenn die Auflagefläche für die Einlage in der Wanne an einer oder mehreren Stellen eine pfeilförmige Vertiefung oder Erhebung aufweist. Dabei weist die Einlage an einer korrespondierenden Stelle vorteilhaft ebenfalls eine entsprechende pfeilförmige Vertiefung oder Erhebung auf, die (im Wesentlichen formschlüssig). So kann die pfeilförmige Vertiefung oder Erhebung in der Einlage (im Wesentlichen passgenau) auf der korrespondierenden pfeilförmigen Vertiefung oder Erhebung in der Auflagefläche der Wanne angeordnet werden. So kann die Stabilität der Einlage in der Wanne weiter erhöht werden. Hierfür kann die Auflagefläche für die Einlage in der Wanne an einer oder mehreren Stellen vorteilhaft verbreitert sein. Besonders vorteilhaft ist die Anordnung einer pfeilförmigen Vertiefung oder Erhebung in einer Ecke der Wanne bzw. Einlage.

So kann eine Ecke der Einlage vorteilhaft eine pfeilförmige Vertiefung (oder eine pfeilförmige Erhebung) aufweisen. Zu diesem Zweck kann die Wanne in der korrespondierenden Ecke eine im Wesentlichen formschlüssig korrespondierende pfeilförmige Vertiefung (oder eine pfeilförmige Erhebung) aufweisen. Dabei zeigt der Kopf des Pfeils der pfeilförmigen Vertiefung (oder Erhebung) der Einlage bzw. der Wanne bevorzugt in diagonaler Richtung zur Ecke (Rand) der Einlage bzw. Wanne. Die Spitze des Pfeilkopfs kann abgerundet oder abgeflacht (d.h. ohne eigentliche "Spitze") ausgeführt werden. Das hintere, breite Ende des Pfeilkopfes ist im Wesentlichen senkrecht zur diagonalen Ausrichtung des Pfeils angeordnet. Die beiden Ränder der Vertiefung (oder Erhebung), die das hintere, breite Ende des Pfeilkopfes bilden, sind daher im Wesentlichen senkrecht zur diagonalen Ausrichtung des Pfeils angeordnet. So kann die Einlage noch besser in der Wanne fixiert werden.

Zu diesem Zweck ist es besonders vorteilhaft, wenn die Einlage und die Wanne jeweils zwei (formschlüssig zwischen Einlage und Wanne korrespondierende) pfeilförmige Vertiefungen (oder Erhebungen), insbesondere in diagonal gegenüber liegenden Ecken der Einlage bzw. Wanne aufweisen. Dabei können die diagonal gegenüber liegenden Ecken der Einlage bzw. Wanne mit pfeilförmiger Vertiefung ferner vorteilhaft zur Anbringung eines Befestigungsmittels, wie anspruchsgemäß definiert, (wie bspw. im Folgenden beschrieben) ausgelegt sein - z.B. durch entsprechende Aufnahmen für eine Verriegelung durch die Befestigungsmittel in der Wanne und durch entsprechende Öffnungen für die Befestigungsmittel in der Einlage. Besonders vorteilhaft ist es, wenn alle vier Ecken der Einlage bzw. Wanne jeweils (formschlüssig zwischen Einlage und Wanne korrespondierende) pfeilförmige Vertiefungen (oder Erhebungen) aufweisen. Auf diese Weise wird selbst bei einem Sturz oder Fall (bspw. während des Transports) verhindert, dass die Einlage in der Wanne verrutscht.

Die Einlage ist reversibel in der Wanne befestigt. Die reversible Befestigung, bspw. mittels einer Verriegelung (z.B. Drehverschluss), der Einlage in der Wanne erfolgt anspruchsgemäß durch ein Befestigungsmittel mit einem Griff und einer Basis. Alternativ (nicht anspruchsgemäß) ist auch offenbart, dass die reversible Befestigung auf unterschiedliche Weise ohne das anspruchsgemäße Befestigungsmittel erfolgen kann mittels Einklicken, Einschieben oder Festziehen (bspw. mit Schrauben als Befestigungsmittel). Offenbart (nicht anspruchsgemäß) ist ein Einschieben oder Einklicken der Einlage in der/die Wanne, bspw. ohne weitere Befestigungsmittel. Hierfür können in der Wanne entsprechende Vertiefungen, Öffnungen, Aussparungen, Fortsätze, oder Nuten vorgesehen sein, die eine reversible Aufnahme der Einlage ermöglichen. Ebenso kann die Einlage (zu den in der Wanne passende) Vertiefungen, Öffnungen, Aussparungen, Fortsätze, oder Nuten zur reversiblen Befestigung in der Wanne aufweisen. Die reversible Befestigung, also das anspruchsgemäße Befestigungsmittel, kann vorteilhaft mit den oben beschriebenen pfeilförmigen Vertiefungen (oder Erhebungen) in Einlage bzw. Wanne kombiniert werden.

Anspruchsgemäß umfasst das erfindungsgemäße Verpackungssystem somit ein Befestigungsmittel zur reversiblen Befestigung der Einlage in der Wanne. Das Befestigungsmittel kann bspw. an der Einlage angebracht sein. Dabei kann das Befestigungsmittel unbeweglich oder beweglich (bspw. drehbar) an der Einlage angebracht sein. Die Verbindung des Befestigungsmittels mit der Einlage kann reversibel oder irreversibel ausgeführt sein. Anspruchsgemäß weist die Einlage Öffnungen auf, um das Befestigungsmittel durch die Einlage hindurchzuführen und/oder um dieses drehbar an der Einlage zu befestigen. Das Befestigungsmittel kann die Einlage bspw. mittels einer Verriegelung (z.B. mittels eines Drehverschlusses) reversibel in der Wanne befestigen.

Das Befestigungsmittel weist anspruchsgemäß einen oberen Teil (einen Griff des Befestigungsmittels) und einen unteren Teil (eine Basis des Befestigungsmittels) auf. Der obere Teil (Griff) weist einen Greifabschnitt und einen Fortsatz zur Verbindung mit der Basis auf, wobei der Fortsatz durch eine Öffnung in der Einlage hindurchgeführt ist. Auf diese Weise können der Greifabschnitt des oberen Teils (Griffs) und der untere Teil (Basis) des Befestigungsmittels (im verbundenen Zustand) auf einander gegenüberliegenden Seiten der Einlage angeordnet sein. Dabei kann der untere Teil (Basis) im Wesentlichen unterhalb der Einlage angeordnet sein (und sich ggf. in oder durch die Öffnung der Einlage nach oben erstrecken). Der obere Teil (Griff) des Befestigungsmittels kann im Wesentlichen oberhalb der Einlage angeordnet sein (und sich ggf. in oder durch die Öffnung der Einlage nach unten erstrecken). Ein kleiner Teilabschnitt des unteren Teils (Basis) des Befestigungsmittels kann auch oberhalb der Einlage angeordnet sein und/oder ein kleiner Teilabschnitt des oberen Teils (Griffs) des Befestigungsmittels kann auch unterhalb der Einlage angeordnet sein, bspw. um beide Teile des Befestigungsmittels miteinander zu verbinden.

So kann das Befestigungsmittel, insbesondere der obere Teil (Griff), mit der Einlage verbunden (bzw. an dieser angebracht) sein. Insbesondere kann der Griff derart ausgelegt sein, dass er neben der Bedienung des Befestigungsmittels auch zur Entnahme der Einlage bzw. zu deren Transport (mit dem Schlauchset und ggf. weiteren verpackten Gegenständen) verwendet werden kann. Bevorzugt ist der Griff daher mit der Einlage verbunden und derart ausgelegt, dass er zur Entnahme und/oder zum Transport der Einlage, insbesondere mit den darauf verpackten Komponenten des Schlauchsets, sowie ggf. mit allen weiteren verpackten Gegenständen, dienen kann.

So weist der obere Teil (Griff) des Befestigungsmittels einen Greifabschnitt und einen Fortsatz zur Verbindung mit dem unteren Teil (Basis) des Befestigungsmittels auf. Der Fortsatz des oberen Teils (Griffs) des Befestigungsmittels ist durch eine Öffnung der Einlage hindurchgeführt (oder in eine solche Öffnung hinein), insbesondere um den oberen Teil (Griff) des Befestigungsmittels auf der Oberseite der Einlage mit dem unteren Teil (Basis) des Befestigungsmittels auf der Unterseite der Einlage zu verbinden. Hierzu kann der untere Teil (Basis) des Befestigungsmittels vorteilhaft eine (passgenaue) Aufnahme für den Fortsatz des oberen Teils des Befestigungsmittels aufweisen. Durch Einbringen des Fortsatzes in die Aufnahme kann eine geeignete Verbindung des oberen und des unteren Teils des Befestigungsmittels erzielt werden.

Eine solche Verbindung des oberen Teils (Griffs) mit dem unteren Teil (Basis) des Befestigungsmittels ermöglicht es in der Regel, dass Bewegungen am oberen Teil (Griff) des Befestigungsmittels, insbesondere an dessen Greifabschnitt ausgeführte Bewegungen - wie bspw. eine Drehbewegung - auf den unteren Teil (Basis) des Befestigungsmittels übertragen werden. Wenn z.B. am Greifabschnitt durch den Benutzer eine Drehbewegung ausgeführt wird, wird damit auch die (unterhalb der Einlage befindliche) Basis des Befestigungsmittels gedreht.

Der obere Teil (Griff) des Befestigungsmittels ermöglicht bspw. die Bedienung des Befestigungsmittels durch den Benutzer. So kann der Benutzer mit dem Griff (Greifabschnitt) die Einlage in der Wanne (z.B. durch manuelle Betätigung des Befestigungsmittels) befestigen oder lösen. Der untere Teil (Basis) des Befestigungsmittels kann bspw. der Verriegelung dienen, etwa im Zusammenwirken mit Vorsprüngen der Innenwand der Wanne. Vorzugsweise weist der untere Teil (Basis) des Befestigungsmittels einen oder mehrere Flügel auf.

Vorzugsweise ist das Befestigungsmittel (in der Einlage) drehbar. Ein (oder mehrere) Flügel der Basis des Befestigungsmittels kann/können durch Drehung des Befestigungsmittels (bspw. durch den Benutzer am Griff) in einer Aufnahme oder unter Vorsprüngen der (Innen-)Wand der Wanne positioniert werden. Dadurch kann eine vertikale Bewegung der Einlage aus der Wanne heraus im Wesentlichen verhindert werden.

Hierfür kann die Wanne vorzugsweise (im bodennahen Bereich, insbesondere im unteren Drittel oder Viertel der Wandhöhe) eine Aufnahme für eine Verriegelung durch das Befestigungselement (Befestigungsmittel) aufweisen. Eine solche Aufnahme kann durch einen oder mehrere entsprechend dimensionierte(n) Vorsprung/Vorsprünge in der (Innen-)Wand der Wanne gebildet werden. Mit anderen Worten, die Wanne kann vorzugsweise (im bodennahen Bereich) einen oder mehrere Vorsprünge an ihrer Innenwand aufweisen, unter dem/denen ein Flügel der Basis des Befestigungsmittels positioniert werden kann.

Vorteilhaft kann die Wanne bspw. zwei Aufnahmen für eine Verriegelung durch zwei Befestigungsmittel, insbesondere mit (bspw. jeweils zwei) Vorsprüngen, aufweisen. Ein solches Verpackungssystem umfasst daher, neben Einlage und Wanne, zwei Befestigungsmittel zur reversiblen Befestigung der Einlage in der Wanne. Dabei sind die Aufnahmen für die beiden Befestigungsmittel in der Wanne (und ggf. entsprechende Öffnungen in der Einlage) möglichst derart voneinander beabstandet, dass der Abstand zwischen den beiden Aufnahmen in der Wanne größer ist als der Abstand von jeder der Aufnahmen jeweils zum nächstgelegenen Rand der Wanne. Insbesondere ist der Abstand zwischen den beiden Aufnahmen in der Wanne mindestens doppelt, bevorzugt mindestens viermal, mehr bevorzugt mindestens sechsmal, noch mehr bevorzugt mindestens achtmal und besonders bevorzugt mindestens zehnmal so groß wie der Abstand von jeder der Aufnahmen jeweils zum nächstgelegenen Rand der Wanne. Bei rechteckiger Grundform der Wanne können bspw. zwei Aufnahmen mit (bspw. jeweils zwei) Vorsprüngen in den diagonal gegenüber liegenden Ecken der Wanne angeordnet sein. Dabei können genau zwei Aufnahmen zur Befestigung der Einlage in der Wanne vorgesehen sein. Vorstellbar sind jedoch bspw. auch vier Aufnahmen zur Befestigung der Einlage in der Wanne, bspw. je eine Aufnahme in jeder Ecke oder je eine Aufnahme an jeder Innenwand (Seitenfläche) der Wanne. Die Aufnahme zur Befestigung der Einlage in der Wanne kann bspw. (genau) einen Vorsprung oder zwei, insbesondere gegenüberliegende, Vorsprünge aufweisen, der/die so dimensioniert ist/sind, dass ein Flügel der unterhalb der Einlage angeordneten Basis des Befestigungsmittels bei Bewegung (bspw. Drehung) des Befestigungsmittels zumindest teilweise unter den/die Vorsprung/Vorsprünge greift. Damit kann die Einlage nicht mehr (oder nur noch begrenzt) nach oben bewegt werden und eine Entnahme der Einlage aus der Wanne kann verhindert werden. Bei entgegengesetzter Bewegung des Befestigungsmittels (bspw. Drehung in Gegenrichtung) greift der Flügel im unteren Teil (bspw. der Basis) des Befestigungsmittels nicht mehr unter den/die Vorsprung/Vorsprünge. Damit wird eine Bewegung der Einlage in vertikaler Richtung (wieder) ermöglicht und die Einlage kann so auf einfache und reversible Weise aus die Wanne herausgenommen bzw. eingesetzt werden.

So bewirken die Vorsprünge auf der Innenwand der Wanne je nach Position des Flügels der Basis des Befestigungsmittels (im Verhältnis zu den Vorsprüngen der Wanneninnenwand) eine Befestigung oder ein Lösen der Einlage in der Wanne. Dabei kann bei Drehung des Befestigungsmittels der Flügel derart gegenüber dem einen oder mehreren Vorsprüngen der Innenwand der Wanne verschoben werden, dass ein Befestigen oder Lösen der Einlage in der Wanne bewirkt wird.

Das Zusammenwirken des/der Vorsprung/Vorsprünge der (Innen-)wand der Wanne mit dem Flügel im unteren Teil (bspw. der Basis) des Befestigungsmittels ermöglicht daher eine Verriegelung durch Bewegung des Flügels relativ zu dem/den Vorsprung/Vorsprüngen. Bei Drehung des Befestigungsmittels zur reversiblen Befestigung der Einlage in der Wanne kann bspw. eine Drehung um etwa 90° erfolgen (insbesondere bei zwei gegenüberliegenden Vorsprüngen), um die Einlage in der Wanne zu befestigen oder zu lösen. Je nach Dimensionierung des Flügels im Vergleich zum Vorsprung können jedoch auch kleinere oder größere Drehwinkel zur Verriegelung bzw. zum Lösen der Verriegelung dienen.

So kann der Benutzer bspw. durch Drehung des Greifabschnitts (des Griffs) einen Flügel (der Basis) des Befestigungsmittels drehen, so dass dieser unter einem Vorsprung der (Innen-)wand positioniert wird (und damit die Einlage in der Wanne befestigt wird). Ebenso kann eine (weitere oder entgegengesetzte) Drehbewegung am Greifabschnitt die Befestigung der Einlage in der Wanne lösen, wenn der Flügel der Basis des Befestigungsmittels nicht mehr unter einem Vorsprüngen der (Innen-)wand der Wanne positioniert ist. Eine derartige Verriegelung kann so ausgeführt werden, dass sie in einer möglichst optimalen Position (bspw. bei größtmöglicher Überlappung von Flügel und Vorsprung; z.B. nach Drehung um ca. 90°) einrastet. Die äußeren Seitenränder des Flügels können eine abgerundete oder abgeschrägte Form aufweisen, bspw. um die Drehbewegung zu erleichtern oder um ein Einrasten der Verriegelung zu ermöglichen oder zu erleichtern.

So kann bspw. in einer bevorzugten Ausführungsform eine Kombination von
(i) einem Vorsprung der (Innen-)wand der Wanne mit einem Flügel im unteren Teil (bspw. der Basis) des Befestigungsmittels (die eine vertikale Bewegung nach oben (aus der Wanne heraus) begrenzen bzw. verhindern); und
(ii) (a) einer Auflagefläche der Wanne (mit dem aufliegenden Randbereich der Einlage), oder
   (b) entsprechend dimensionierten Vertiefungen in der Wanne (die eine vertikale Bewegung der Einlage nach unten (in die Wanne hinein) begrenzen bzw. verhindern)
vorteilhaft eine Bewegung der Einlage in vertikaler Richtung insgesamt minimieren bzw. im Wesentlichen verhindern.

Der Greifabschnitt des oberen Teils (bspw. eines Griffs) des Befestigungsmittels kann verschiedene Formen aufweisen. Dabei kann der Greifabschnitt vorteilhaft an die auszuführende Bewegung zur Befestigung angepasst sein. Bspw. sind für manuelle Drehbewegungen Greifabschnitte einer gewissen Breite, die gut mit einer Hand bedient werden können, von Vorteil.

Um eine möglichst einfache Bedienung und leichte Erreichbarkeit das Greifabschnitts zu ermöglichen, kann der oberhalb der Einlage befindliche obere Teil des Befestigungsmittels so dimensioniert werden, dass der Greifabschnitt in einem oberen Abschnitt der Wanne angeordnet ist, vorzugsweise möglichst kurz unterhalb des oberen Wannenrands. Bspw. kann der oberhalb der Einlage befindliche obere Teil des Befestigungsmittels so dimensioniert sein, dass die Oberseite des Griffs des Befestigungsmittels den oberen Wannenrand gerade so nicht überragt (so dass bei befestigter Einlage eine Versiegelung der Wanne ohne Einschränkungen möglich ist). Alternativ kann der Greifabschnitt auch in einem mittleren oder unteren Abschnitt der Wanne vorgesehen sein.

Das Befestigungsmittel, insbesondere dessen oberer Teil (Griff), kann in einer bevorzugten Ausführungsform einen Haken enthalten, der bei Bedarf aus dem Befestigungsmittel heraus gelöst (bspw. herausgebrochen) werden kann.

So stellt die Erfindung gemäß Anspruch 19 in einem weiteren Aspekt auch ein Befestigungsmittel zur reversiblen Befestigung einer Einlage in einer Wanne bereit, das einen Griff und eine Basis umfasst, wobei der Griff einen Greifabschnitt und einen Fortsatz zur Verbindung mit der Basis aufweist, der durch eine Öffnung in der Einlage hindurchgeführt werden kann. Im Griff kann ein Haken integriert sein, der aus dem Griff heraus gelöst werden kann. Die detaillierte Beschreibung des Befestigungsmittels im Kontext des erfindungsgemäßen Verpackungssystems ist daher auch auf das erfindungsgemäße Befestigungsmittel anwendbar und umgekehrt.

Der Haken im oberen Teil (Griff) des Befestigungsmittels kann eine Halterung zur Befestigung des Hakens bspw. an einem Stab oder an einer Schiene aufweisen. So weisen im medizinischen Therapiebereich anzutreffende Schienen oder Stäbe (z.B. an der Konsole, wo auch der Oxygenator und/oder ein Elektromotor zum Antrieb der Blutpumpe des Schlauchsets im Betrieb am Patienten angeordnet werden können) häufig Dimensionen nach DIN-Norm auf, so dass eine entsprechend darauf abgestimmte Halterung, und damit der Haken, in diesem Bereich breit einsetzbar ist. Die Halterung kann bspw. so ausgeführt sein, dass der Haken auf einfache Weise an einen Stab bzw. an eine Schiene geklippt werden kann.

Durch das Herauslösen des Hakens aus dem Befestigungsmittel werden insbesondere weder das Befestigungsmittel noch der Haken beschädigt und/oder weder die Funktionalität des Befestigungsmittels noch die Funktionalität des Hakens beeinträchtigt. Hierzu kann der Haken lediglich über wenige (bspw. zwei oder drei) möglichst dünne Verbindungsstege im Befestigungsmittel integriert sein. Die Verbindungsstege können bspw. Sollbruchstellen aufweisen, z.B. indem bestimmte Abschnitte der Verbindungsstege besonders dünn ausgeführt werden (dünner als der restliche Verbindungssteg). Der obere Teil des Befestigungsmittels mit dem integrierten Haken kann vorteilhaft einstückig ausgeführt sein, so dass der obere Teil mit dem integrierten Haken im selben Arbeitsgang hergestellt werden kann (und keine zusätzlichen Arbeitsgänge zur Integration des Hakens im Befestigungsmittel erforderlich sind). Daher besteht der Haken in der Regel aus demselben Material wie das Befestigungsmittel bzw. dessen oberer Teil.

Das Material des Befestigungsmittels ist nicht eingeschränkt, solange die Funktionalität des Befestigungsmittels gewährleistet wird und das Material sterilisierbar ist. Der obere Teil (Griff) und der untere Teil (Basis) des Befestigungsmittels können aus demselben Material oder aus unterschiedlichen Materialien bestehen. Vorteilhafte Materialien sind Kunststoffe, wie bspw. Polypropylen und PBT (Polybutylenterephthalat). PBT ist als Material für den unteren Teil des Befestigungsmittels (Basis) oder für das gesamte Befestigungsmittel bevorzugt, da durch die im Material integrierten Fasern die Stabilität vorteilhaft erhöht wird. Der obere Teil des Befestigungsmittels (Griff) kann auch vorteilhaft aus Polypropylen bestehen, um ein Ausbrechen des ggf. vorhandenen Hakens aus dem Griff zu erleichtern.

Das Befestigungsmittel kann auch dazu verwendet werden, die Einlage (außerhalb der Wanne) - und damit ggf. die darauf/darin verpackten Gegenstände sowie ggf. weitere auf der Einlage befindliche Elemente des Verpackungssystems - zu transportieren, bspw. auf der Intensivstation. Ein solcher Transport der Einlage am Befestigungsmittel (mit den verpackten Gegenständen) kann bspw. bei der Verpackung geschehen, wenn das Schlauchsystem vorteilhaft zunächst außerhalb der Wanne auf bzw. in der Einlage verpackt wird, so dass danach nur noch die Einlage in die Wanne verbracht und die Einlage mit dem daran befindlichen Befestigungsmittel in der Wanne befestigt werden muss. Ebenso kann beim Auspacken (bspw. auf der Intensivstation) zunächst die Befestigung der Einlage in der Wanne gelöst werden und dann die Einlage mit den darauf/darin befindlichen Gegenständen der Wanne entnommen werden und an einen Zielort zum (weiteren) Auspacken verbracht werden. Die Einlage mit dem einen oder mehreren Befestigungsmittel(n) eignet sich daher auch zum sicheren Transport des Schlauchsets (ohne Wanne), bspw. innerhalb steriler Bereiche. Auf diese Weise kann eine Berührung des Schlauchsets (und damit eine mögliche Kontamination) während des Transports (bspw. an den Zielort auf der Intensivstation) vermieden werden. So ist die Einlage vorteilhaft derart ausgelegt, dass alle Komponenten des Schlauchsets, sowie ggf. alle weiteren zu verpackenden Gegenstände, (vollständig) auf (d.h. oberhalb) der Einlage verpackt werden können.

Ferner kann das erfindungsgemäße Verpackungssystem eine oder mehrere Abdeckung(en) für eine (oder mehrere) Vertiefung(en) oder einen (oder mehrere) Aufnahmeabschnitt(e) in der Einlage aufweisen. Die Abdeckung kann die Vertiefung oder den Aufnahmeabschnitt, insbesondere mit der/den darin/darauf verpackten Schlauchsetkomponente(n) bspw. im Wesentlichen formschlüssig abdecken. So können auf modulare Weise unterschiedliche Kompartimente im Verpackungssystem geschaffen werden. Damit wird sowohl eine geordnete Verpackung (so dass sich bestimmte Schlauchset-Komponenten während des Transports nicht berühren und daher nicht gegenseitig beschädigen können); eine übersichtliche Anordnung der verpackten Schlauchset-Komponenten sowie ein Auspacken/eine Entnahme des Schlauchsets in der Reihenfolge der Verwendung ermöglicht. So sind zunächst die auf (oberhalb) einer Abdeckung verpackten verpackte Gegenstände sichtbar und werden daher beim Auspacken in der Regel zuerst dem Verpackungssystem entnommen, während unter einer Abdeckung verpackte Gegenstände erst später ausgepackt werden (nach Entfernen der Abdeckung). So kann der Benutzer bei entsprechender Verpackung das verpackte Schlauchset intuitiv schrittweise in der Reihenfolge der Verwendung entnehmen.

Die Abdeckung kann ihrerseits eine oder mehrere Vertiefung(en) oder Aufnahmeabschnitt(e) zur Aufnahme von Komponenten des Schlauchsets oder zusätzlichen Gegenständen, die im Zusammenhang mit dem medizinischen Schlauchset verwendet werden können, wie bspw. Schlauchklemmen, aufweisen. Auf diese Weise können Komponenten des Schlauchsets (oder zusätzliche Gegenstände) im erfindungsgemäßen Verpackungssystem (durch die Abdeckung voneinander getrennt) übereinander angeordnet werden, ohne sich gegenseitig zu beschädigen. Diese Vertiefung(en) oder Aufnahmeabschnitt(e) können bspw. zur im Wesentlichen formschlüssigen) Aufnahme ausgebildet sein, bspw. indem sie die Form des aufzunehmenden Gegenstandes im Wesentlichen widerspiegeln.

Beispielsweise kann das Verpackungssystem eine (im Wesentlichen formschlüssige) Abdeckung für eine Vertiefung in der Einlage aufweisen, die zur Abdeckung eines in einer Vertiefung der Einlage gelagerten Gegenstands, bspw. eines Schlauchs, dienen kann. Eine solche Abdeckung kann ihrerseits eine oder mehrere Vertiefung(en) zur im Wesentlichen formschlüssigen Aufnahme bzw. Anordnung von Komponenten des Schlauchsets oder von zusätzlichen Gegenständen, die im Zusammenhang mit dem medizinischen Schlauchset verwendet werden können, aufweisen, wie bspw. zur (im Wesentlichen formschlüssigen) Aufnahme von Schlauchklemmen.

Eine weitere Abdeckung kann bspw. als Abdeckung für die erste Abdeckung ausgelegt sein. So kann die erste Abdeckung zur Abdeckung eines in einer Vertiefung der Einlage gelagerten Gegenstands, bspw. eines Schlauchs, dienen und gleichzeitig können Komponenten des Schlauchsets oder zusätzlichen Gegenstände auf der ersten Abdeckung angeordnet sein, wie oben beschrieben. Die weitere Abdeckung dient dann zur Abdeckung der auf der ersten Abdeckung gelagerten Komponenten bzw. Gegenstände. Auf diese Weise können mehrere Abdeckungen platzsparend übereinander (gestapelt) angeordnet werden, wobei vorteilhaft Komponenten des Schlauchsets bzw. zusätzliche Gegenstände voneinander getrennt auf übersichtliche Weise verpackt werden können. Dabei kann die zuoberst angeordnete Abdeckung bspw. eine Führung für ein Fixierungsmittel (bspw. ein Band) aufweisen, das zur Fixierung von Komponenten des Schlauchsets (oder weiterer Gegenstände, die verpackt werden sollen) auf der Einlage und/oder auf einer (der mehreren) Abdeckung(en) dient. So kann mit Hilfe des Fixierungsmittels die (oberste) Abdeckung - sowie ggf. darunter befindliche Abdeckung(en) - und darin gelagerte Gegenstände fixiert werden.

Auch die weitere Abdeckung kann ihrerseits eine oder mehrere Vertiefung(en) bzw. Erhebungen zur im Wesentlichen formschlüssigen Aufnahme bzw. Anordnung von Komponenten des Schlauchsets oder von zusätzlichen Gegenständen, die im Zusammenhang mit dem medizinischen Schlauchset verwendet werden können, aufweisen. Beispielsweise kann die (weitere bzw. oberste) Abdeckung Erhebungen zur Aufnahme/Anordnung einer Spritze aufweisen. Zudem kann die Führung auf einer (beispielsweise im Wesentlichen kreisrunden oder elliptischen) Erhebung angeordnet sein, die ihrerseits die Aufnahme bzw. Anordnung eines (aufgerollten) Schlauchs (um die Erhebung herum) ermöglicht. Dabei sind die Vertiefungen oder Erhebungen zur Aufnahme von Komponenten des Schlauchsets oder von zusätzlichen Gegenständen vorteilhaft derart angeordnet, dass darin angeordnete Gegenstände ebenfalls durch das in der Führung geführte Fixierungsmittel zusätzlich fixiert werden können.

Ferner kann in einigen Ausführungsformen des erfindungsgemäßen Verpackungssystems eine oder mehrere der Vertiefungen in der Einlage zumindest an einer Seite der Vertiefung eine seitliche Aussparung aufweisen. Eine solche Vertiefung ist selbst bei (formschlüssiger) Abdeckung mit einer oben beschriebenen Abdeckung seitlich nicht vollständig abgeschlossen. Auf diese Weise können bspw. ein oder mehrere Schläuche seitlich in die Vertiefung hinein- oder herausgeführt werden. In der seitlichen Aussparung der Vertiefung kann (vorzugsweise formschlüssig) ein Schott angeordnet sein, durch das ein Gegenstand (Komponente des Schlauchsets, insbesondere ein Schlauch), insbesondere kraft- oder formschlüssig (vorzugsweise kraftschlüssig), hindurchgeführt werden kann. Dabei kann die Vertiefung mit Abdeckung und Schott ein besonders geschütztes Kompartiment (abgedeckter Innenraum der Vertiefung) ausbilden, der bspw. bis auf die durch das Schott hinein- bzw. herausgeführten Gegenstände (wie Schläuche) abgeschlossen ist. Ein solches Kompartiment ist besonders vor Kontaminationen geschützt.

So können eine oder mehrere Vertiefungen in der Einlage dafür vorgesehen sein, mittels einer Abdeckung und geeigneten Mitteln zur Abdichtung (wie bspw. einem Schott) besonders geschützte Kompartimente im Verpackungssystem auszubilden. Solche besonders geschützten Kompartimenten können bspw. der Unterbringung offener Enden eines (blutführenden) Schlauchs dienen, die besonders vor Kontaminationen (bspw. durch kleinste Staubpartikel) geschützt werden müssen. Um einen Schlauch in ein solches besonders geschütztes Kompartiment hinein- oder herauszuführen, können in seitlichen Aussparungen der Vertiefung ein oder mehrere Schotts angeordnet sein.

Das Verpackungssystem kann daher ferner ein (oder mehrere) Schott(s) umfassen, das eine oder mehrere Öffnungen für eine (möglichst passgenaue) Durchführung von Schläuchen aufweisen kann. Insbesondere kann das Schott eine äußerliche Formgebung (Umriss) aufweisen, die eine seitliche Aussparung einer Vertiefung (in der Einlage) formschlüssig ausfüllen kann. Auf diese Weise kann mittels Schotts in einer Vertiefung der Einlage mit der Abdeckung ein möglichst abgeschlossener Innenraum gebildet werden, der vor Kontaminationen besonders geschützt ist und in den dennoch mittels der Öffnungen der Schotts ein oder mehrere Schläuche (im Wesentlichen kraftschlüssig) hinein- oder hinausgeführt werden können. Darüber hinaus können die Schotts der Arretierung der Schläuche im Verpackungssystem dienen (wobei hierfür die äußerliche Formgebung in der Regel unerheblich ist).

In der Regel kann genau ein Schlauch durch genau eine Öffnung eines Schotts geführt werden. Die eine oder mehreren Öffnung(en) in dem Schott können genau an den Schlauchumfang (Schlauchgröße) angepasst sein. Dadurch werden Kontaminationen des gebildeten Kompartiments (Innenraums der Vertiefung) vermieden. So kann ein Verpackungssystem ein oder mehrere Schotts umfassen, wobei alle Öffnungen des Schotts im Wesentlichen identisch sind (und daher für dieselbe Schlauchgröße, bspw. eines blutführenden Schlauchs, ausgelegt sind). Alternativ kann ein Verpackungssystem auch ein oder mehrere Schotts enthalten, die (unterschiedliche) Öffnungen für unterschiedliche Schlauchgrößen aufweisen. Bspw. kann ein Schott 1, 2, 3, 4, 5 oder mehr Öffnungen ausweisen. Vorzugsweise weist ein Schott (genau) eine oder zwei Öffnungen auf. Wenn ein Schott Öffnungen für mehrere Schläuche aufweist, können alle Öffnungen des Schotts für dieselbe oder für verschiedene Schlauchgrößen (Schlauchumfänge) ausgelegt sein. Häufige beispielhafte Schlauchgrößen im medizinischen Bereich sind 1/4" oder 3/8", für die das Schott entsprechende Öffnungen (Öffnungen der Größe 1/4" oder 3/8") aufweisen kann. Um Verwechslungen der Schotts für verschiedene Schlauchgrößen zu vermeiden, kann das Schott eine Markierung entsprechend der Schlauchgröße aufweisen. So kann bspw. die Schlauchgröße auf das Schott aufgebracht werden, z.B. mittels Spritzgusstechnik, so dass sie bspw. bei geschlossenem Schott sichtbar ist.

Zur (kraftschlüssigen) Einbringung der Schläuche in ein Schott ist es vorteilhaft, wenn das Schott geöffnet (aufgeklappt) werden kann, bspw. in zwei (Längs-)Teile (z.B. (Längs-)Hälften) des Schotts. Dabei verläuft die Achse zum Öffnen/Aufklappen des Schotts vorzugsweise (im Wesentlichen zentral) durch die eine oder mehreren für die Schläuche vorgesehene(n) Öffnung(en) des Schotts. So kann der Schlauch bei geöffnetem (aufgeklappten) Schott einfach (in einen Teil des Schotts bzw. in einen Teil der (geöffneten) Öffnung des Schotts) eingelegt werden und das Schott dann (mit eingelegtem Schlauch) verschlossen werden. Ein Kraft- bzw. Formschluss zwischen eingelegtem Schlauch und Öffnung im Schott entsteht dann ggf. erst mit Verschluss (Zusammenklappen/Schließen) des Schotts. Bspw. kann das Schott ein Gelenk oder Scharnier aufweisen. Damit können die zwei (Längs-)Teile ((Längs-)Hälften) des Schotts auf und zugeklappt werden. Auf diese Weise können die Schläuche einfach in ein offenen Schotts eingelegt werden und die Schotts dann verschlossen werden. Besonders vorteilhaft kann ein Schott einen Labyrinthverschluss aufweisen. Dieser kann mehrere Querstege aufweisen. So ermöglicht ein Labyrinthverschluss einen besonders dichten Verschluss des Schotts.

Das Schott kann vorteilhaft gasdurchlässig (im Bereich außerhalb der Öffnungen) gestaltet sein. Auf diese Weise kann eine Sterilisation mittels Gas, bspw. durch Ethylenoxid-Gas, in der Verpackung ermöglicht bzw. erleichtert werden. So kann einerseits eine besonders sichere Fixierung des Schlauches in seiner Position gewährleistet werden, ohne andererseits die Be- und Ausgasung zu beeinflussen.

Das (geschlossene) Schott mit dem darin eingelegten Schlauch bzw. den darin eingelegten Schläuchen kann dann (vorzugsweise formschlüssig) in eine seitliche Aussparungen einer Vertiefung der Einlage eingesetzt werden. Dies kann z.B. durch Einklicken des Schotts in die Einlage erfolgen.

Ferner kann das Verpackungssystem Fixierungsmittel umfassen, mit denen Komponenten des Schlauchsystems im Verpackungssystem fixiert werden können (bspw. auf/in/an der Einlage oder einer Abdeckung). Hierfür kann ein Schott, wie oben beschrieben, verwendet werden. Weitere nicht einschränkende Beispiele für Fixierungsmittel sind ein Band (bzw. mehrere Bänder) und ein (oder mehrere) Fixierclip(s).

Das Verpackungssystem kann vorteilhaft ein Band oder mehrere (bspw. zwei) Bänder umfassen. Das Band kann der Fixierung von Komponenten des Schlauchsets (oder weiterer Gegenstände, die verpackt werden sollen) auf der Einlage und/oder auf einer (der mehreren) Abdeckung(en) dienen. Es kann ebenfalls der Fixierung einer Abdeckung auf einer Vertiefung oder einem Aufnahmeabschnitt (mit Schlauchsetkomponenten) dienen.

Das Band bzw. die Bänder ist/sind vorzugsweise elastisch. Dem Fachmann sind verschiedene geeignete elastische Materialien bekannt, bspw. Silikon oder Gummi. Silikone umfassen synthetischer Polymere, bei denen Siliziumatome über Sauerstoffatome verknüpft sind (Poly(organo)siloxane). Gummis umfassen Vulkanisate von Natur- und Synthesekautschuken. Bevorzugt ist das Band aus Silikon. Ein elastisches Band ist vielseitig einsetzbar und ermöglicht ein einfaches und sicheres Verpacken des Schlauchsets. So können mit demselben Band Gegenstände (bspw. Oxygenatoren) unterschiedlicher Größe fixiert werden. Auf diese Weise können dieselben Bänder in Verpackungssystemen für verschiedene Schlauchsets eingesetzt werden, d.h. die Bänder müssen nicht für jedes Verpackungssystem separat konfektioniert werden. Ein elastisches Material kann außerdem eine zusätzliche Sicherheit beim Transport und der Lagerung empfindlicher Gegenstände (wie Oxygenatoren) gewährleisten, da hierdurch der empfindliche Gegenstand (bspw. ein Oxygenator) in der Einlage abgefedert und sicher gehalten wird, so dass abrasiver Verschleiß und Kratzer durch Vibrationen vermieden werden können. Zudem ermöglicht ein elastisches Band ein einfaches Ver- und Entriegeln.

Das Band bzw. die Bänder können auf einer Seite (d.h. an einem Ende des Bands) an oder in der Einlage fixiert sein. Auf der anderen Seite (am anderen Ende) kann das Band derart an der Einlage befestigt sein (bspw. mit einem definierten Anzugsmoment), dass es leicht gelöst und bei Bedarf wieder befestigt werden kann. Bspw. kann die andere Seite (das andere Ende) des Bands einen (reversiblen) Verschluss an der Einlage bilden, der sich bspw. leicht öffnen und schließen lässt.

Das Band weist zwei Endbereiche auf (jeweils einen an den beiden Enden des Bandes), wobei ein "Endbereich" der (jeweils) terminale (am Ende befindliche) Bereich des Bandes ist, der etwa ein Drittel (bevorzugt etwa ein Viertel, mehr bevorzugt etwa ein Fünftel) der Gesamtlänge des Bandes umfasst. So umfassen die beiden Endbereiche des Bandes bspw. die beiden terminalen Drittel (bevorzugt die beiden terminalen Viertel, mehr bevorzugt die beiden terminalen Fünftel) des Bandes (bezogen auf die Gesamtlänge des Bandes).

Das Band kann in einem Endbereich eine oder mehrere Öffnungen aufweisen und im anderen Endbereich einen Bereich (beidseitig) verringerter Breite, auf den zum (Band-)Ende hin ein verbreiterter Bereich folgt (der in der Regel das Ende des Bandes bildet). Dies ermöglicht eine einfache Befestigung (ohne weitere Zusatzbauteile) und eine schnelle Entriegelung des Bandes (bzw. damit fixierter Gegenstände. Dabei ist der verbreiterte Bereich (an dem einen Ende des Bandes) breiter als der benachbarte Bereich (beidseitig) verringerter Breite, bspw. mindestens 1,5mal oder doppelt so breit. Außerdem ist der verbreiterte Bereich (an dem einen Ende des Bandes) breiter als die eine oder mehreren Öffnungen im anderen Endbereich des Bandes, bspw. mindestens 1,5mal oder doppelt so breit. Der Bereich (beidseitig) verringerter Breite kann bspw. eine Breite aufweisen, die geringer oder gleich groß wie die Breite der einen oder mehreren Öffnungen im anderen Endbereich des Bandes ist.

Die eine oder mehreren Öffnungen weisen vorzugsweise eine geringere Breite als Länge auf (wobei die Begriffe "Breite" und "Länge" der Öffnungen analog zum Band verwendet werden, d.h. die "Länge" der Öffnungen wird in derselben Richtung gemessen wie die Länge des Bandes (Längsrichtung des Bandes) und die "Breite" der Öffnungen in derselben Richtung wie die Breite des Bandes). Bspw. können die Öffnungen mindestens 1,5 mal oder doppelt so lang wie breit sein. So sind die Öffnungen sind vorzugsweise länglich. Mehrere Öffnungen sind bevorzugt hintereinander (in Längsrichtung des Bandes) angeordnet. Mehrere Abstände zwischen mehreren Öffnungen können in etwa identisch sein. Mehrere Öffnungen haben den Vorteil, dass eine größere Flexibilität bzgl. der Befestigung von Gegenständen unterschiedlicher Größe ermöglicht wird. Bspw. kann das Band 1, 2, 3, 4, 5, 6, 7, 8, 9, 10 oder mehr Öffnungen aufweisen. In bestimmten Ausführungsformen sind die Öffnungen nicht auf den Endbereich des Bandes beschränkt. Bspw. können die Öffnungen über die gesamte Länge des Bandes (bspw. außer dem Bereich (beidseitig) verringerter Breite und ggf. des darauffolgenden Endes) angeordnet sein.

Zum Verschließen (Befestigen) des Bandes kann der Endbereich mit dem verbreiterten Bereich durch eine der Öffnungen im anderen Endbereich hindurchgeführt werden, bspw. bis sich das Band nach dem Bereich (beidseitig) verringerter Breite wieder verbreitert (so dass der Bereich (beidseitig) verringerter Breite im Wesentlichen in der Öffnung verbleibt). Das Hindurchführen kann bspw. ermöglicht werden, indem der verbreiterte Bereich, der benachbart zum Bereich (beidseitig) verringerter Breite angeordnet ist, gedreht wird (bspw. um ca. 90°). Nach dem Hindurchführen kann der verbreiterte Bereich, der benachbart zum Bereich (beidseitig) verringerter Breite angeordnet ist, zurück in dessen ursprüngliche Position gedreht werden (oder dreht sich, bspw. bei elastischer Ausführung des Bandes, selbst in die Ausgangsposition zurück). Dabei verhindert der verbreiterte Bereich an dem einen Ende des Bandes ein unbeabsichtigtes bzw. selbstständiges Öffnen des Bandes (Herausrutschen des Bandendes aus der Öffnung, durch die es hindurchgeführt wurde).

Zum reversiblen Öffnen des Bandes kann wiederum der verbreiterte Bereich durch eine der Öffnungen im anderen Endbereich hindurchgeführt werden (bspw. indem dieser gedreht wird, z. B. um ca. 90°), wobei hier das Hindurchführen in entgegengesetzter Richtung zum Hindurchführen beim Verschließen ausgeführt wird.

Zum leichteren Hindurchführen des verbreiterten Bereichs können dessen Seitenränder eine abgerundete oder abgeschrägte Form aufweisen. So kann der verbreiterte Bereich des Bands bspw. eine im Wesentlichen sechseckige Form aufweisen.

Die Einlage (bzw. die Abdeckung) kann eine oder mehrere (vorzugsweise (genau) zwei pro Band) Öffnungen aufweisen, durch die das Band hindurchgeführt werden kann. Diese Öffnungen in der Einlage dienen zur Fixierung von Gegenständen in bzw. an (der Vertiefung/dem Aufnahmeabschnitt) der Einlage oder Abdeckung mit dem Band. Die Öffnungen in der Einlage (bzw. Abdeckung) sind vorzugsweise mindestens so lang wie das Band breit ist und/oder mindestens so breit wie das Band dick ist.

Um ein horizontales Verrutschen des Bandes (bspw. während des Transports oder der Lagerung) zu verhindern oder zu minimieren, kann in einer Abdeckung eine Führung für das Band vorgesehen sein. So kann das Verpackungssystem eine Abdeckung für eine Vertiefung/einen Aufnahmeabschnitt in der Einlage und/oder für eine Vertiefung/einen Aufnahmeabschnitt in einer weiteren Abdeckung aufweisen, die eine Führung für ein Band aufweist. Auf diese Weise kann das Band gegen ein unbeabsichtigtes horizontales Verschieben gesichert sein. Eine solche Abdeckung kann bspw. (im Wesentlichen formschlüssig) auf der Einlage oder auf einer (weiteren) Abdeckung (bspw. einer Abdeckung mit Vertiefungen zur Aufnahme von zusätzlichen Gegenständen, die im Zusammenhang mit dem medizinischen Schlauchset verwendet werden können) angeordnet sein.

Das Band kann auch verwendet werden, um die Einlage vorteilhaft aufzuhängen. Hierfür kann das (elastische) Band auf einer Seite (zug)fest in der Einlage verankert sein. Auf ähnliche Weise können bspw. auch Gegenstände wie der Oxygenator mittels Band an der Einlage befestigt sein. Nach Entnahme der Gegenstände kann die Einlage an dem (elastischen) Band aufgehängt werden - bspw. an einem Halter der Konsole (dem Steuergerät für die extrakorporale Therapie) bzw. an einem Infusionshaken. Insbesondere wenn die Einlage aufgehängt wird, kann bei Bedarf zumindest ein Teil des (blutführenden) Schlauches während der Verwendung (bspw. während der Vorbereitung und/oder Durchführung der extrakorporalen Therapie) in der Einlage verbleiben. Alternativ kann der (blutführende) Schlauch 20 zur Verwendung auch aus der Einlage entnommen werden.

Ferner kann das Verpackungssystem vorteilhaft einen Fixierclip umfassen. Der Fixierclip ist vorzugsweise so ausgebildet, dass er eine Komponente des Schlauchsets (oder einen Teil einer solchen Komponente) zumindest teilweise umschließt (bspw. kraft- oder formschlüssig). So kann der Fixierclip zum Einsatz an Komponenten mit im Wesentlichen runden Querschnitten/Formen bspw. eine entsprechende Rundung aufweisen, die die im Wesentlichen runde Form/Querschnitt des zu fixierenden Gegenstands zumindest teilweise umschließt. Bspw. kann dabei die Rundung des Fixierclips mehr als die Hälfte (bspw. etwa zwei Drittel) der im Wesentlichen runden Form/Querschnitt des zu fixierenden Gegenstands umschließen. So kann der zu fixierende Gegenstand im Fixierclip fixiert werden. Zudem kann der zu fixierende Gegenstand einfach in den Fixierclip hinein geführt (und von diesem aufgenommen) werden. Der Clip kann vorteilhaft auch für unterschiedliche Schlauchdurchmesser (bspw. für zwei unterschiedliche Schlauchdurchmesser) angelegt sein. Der Fixierclip kann zudem im unteren Bereich Fortsätze aufweisen, mittels derer er in entsprechenden Öffnungen in der Einlage (oder in einer Abdeckung) befestigt werden kann. Dementsprechend kann die Einlage (bzw. eine Abdeckung) Öffnungen zur Befestigung eines Fixierclips aufweisen. Der Fixierclip kann bspw. durch Zusammendrücken des Clips in der Einlage befestigt und anschließend wieder entfernt werden. Der Fixierclip ist bevorzugt aus Kunststoff, bspw. Polypropylen.

Das Verpackungssystem kann vorteilhaft ein Auspacken im Wesentlichen in der Reihenfolge der Verwendung der verpackten Gegenstände ermöglichen. Mit anderen Worten, die Anordnung des Schlauchsets im Verpackungssystem kann vorteilhaft in einer Weise erfolgen, die ein Auspacken im Wesentlichen in der Reihenfolge der Verwendung (bspw. in der Reihenfolge des Aufbaus bzw. der Bereitstellung des Schlauchsets für die extrakorporale Therapie) ermöglicht. Insbesondere kann das Verpackungssystem eine Verpackung ermöglichen, bei der der Benutzer jeweils nur diejenigen Gegenstände sieht, die im nächsten Schritt der Verwendung benötigt werden. Damit können Fehler beim Aufbau und/oder bei der Verwendung der verpackten Gegenstände vermieden werden (Poka-Yoke-Prinzip).

Hierfür können die Komponenten des Schlauchsets vorteilhaft derart verpackt werden, dass solche Komponenten, die zuerst verwendet werden, als letztes verpackt (z.B. möglichst zuoberst) und als erstes entpackt werden können (bspw. durch eine obenauf liegende Position in der Verpackung). Hingegen können Komponenten des Schlauchsets, die später (bzw. zuletzt) verwendet werden, weiter unten (bzw. zuunterst verpackt) werden. Bspw. können durch eine oder mehrere Abdeckungen im Verpackungssystem unterschiedliche Kompartimente geschaffen werden, wobei zunächst die auf (oberhalb) einer Abdeckung verpackten Gegenstände sichtbar sind und daher beim Auspacken in der Regel zuerst dem Verpackungssystem entnommen werden. Hingegen werden unter einer Abdeckung verpackte Gegenstände intuitiv erst später ausgepackt (nach Entfernen der Abdeckung und darauf befindlicher Gegenstände). So kann der Benutzer bei entsprechender Verpackung das verpackte Schlauchset intuitiv schrittweise in der Reihenfolge der Verwendung entnehmen.

Bspw. kann die Einlage Vertiefungen zur Aufnahme eines blutführenden Schlauches (d.h. eines Schlauches zur Blutführung) aufweisen. Aufgrund des Sterilisationsprozesses werden solche Schläuche in der Regel (an ihren Enden) offen geliefert und müssen am Ort der Verwendung entsprechend angeschlossen werden, bspw. an einem Oxygenator. Daher ist es vorteilhaft, Schläuche, in denen Blut des Patienten geführt werden soll, selbst in sterilen Bereichen besonders vor Verunreinigungen, wie kleinsten Staubpartikeln, zu schützen, da das darin geführte Blut (und damit mögliche Verunreinigungen) dem Patienten wieder zugeführt werden. Um lange Zeiträume zwischen der Entnahme von blutführenden Schläuchen aus dem Verpackungssystem und deren Verwendung (und damit eine steigende Wahrscheinlichkeit von Kontaminationen) zu vermeiden, ist es vorteilhaft, beim Auspacken des Schlauchsets blutführende Schläuche möglichst zuletzt (oder als eine der letzten Schlauchsetkomponenten) auszupacken, so dass diese direkt angeschlossen (und für die extrakorporale Therapie vorbereitet) werden können. Daher ist es vorteilhaft, wenn die Einlage entsprechende Vertiefungen zur Aufnahme eines blutführenden Schlauches aufweist, so dass dadurch die "unterste" oder zumindest eine der unteren Verpackungsebenen gebildet wird.

In einem weiteren Aspekt stellt die vorliegende Erfindung stellt auch die Verwendung des anspruchsgemäßen Verpackungssystems zum Verpacken eines medizinischen Schlauchsets bereit. Wie oben beschrieben ist das erfindungsgemäße Verpackungssystem auf diesen Zweck ausgerichtet, so dass detaillierte und bevorzugte Merkmale des anspruchsgemäßen Verpackungssystems ebenso auf dessen Verwendung zur Verpackung eines medizinischen Schlauchsets anwendbar sind.

In einem weiteren Aspekt stellt die Erfindung ein Verfahren zum Verpacken eines medizinischen Schlauchsets in einem Verpackungssystems, wie anspruchsgemäß definiert, bereit, wobei das Verfahren die folgenden Schritte umfasst:
(i) Verpacken des medizinischen Schlauchsets auf der Einlage außerhalb der Wanne;
(ii) Einbringen der Einlage mit dem verpackten Schlauchset in die Wanne; und
(iii) reversible Befestigung der Einlage in der Wanne.

Die detaillierte Beschreibung des erfindungsgemäßen Verpackungssystems ist ebenso auf das erfindungsgemäße Verfahren anwendbar. Insbesondere ermöglicht das erfindungsgemäße Verpackungssystem ein Verpacken eines medizinischen Schlauchsets mit dem erfindungsgemäßen Verfahren, wobei insbesondere das Schlauchset auf einfache Weise außerhalb der Wanne auf der Einlage verpackt werden kann und danach in die Wanne eingebracht und dort reversibel befestigt wird.

So kann Schritt (i) des erfindungsgemäßen Verfahrens mehrere Unterschritte aufweisen, wie bspw. die Befestigung eines Befestigungsmittels an der Einlage. So wird bei einem mehrteiligen Befestigungsmittel (bspw. mit einem oberen und einem unteren Teil) einer der Teile (bspw. der obere Teil) durch eine hierfür in der Einlage vorgesehene Öffnung geführt und kann dann mit dem anderen Teil (bspw. dem unteren Teil) verbunden werden (bspw. durch Einrasten bzw. durch eine Verbindung eines Fortsatzes eines Teils des Befestigungsmittels mit einer dafür vorgesehenen Aufnahme im anderen Teil des Befestigungsmittels).

Weiterhin kann Schritt (i) optional einen Unterschritt der Bestückung von Abdeckungen, soweit vorgesehen, bspw. mit den jeweils hierfür vorgesehenen Komponenten (z.B. zusätzliche Artikel, die in der Regel zusammen mit dem medizinischen Schlauchset verwendet werden, wie bspw. Schlauchklemmen, eine Spritze, Kabelbinder etc.).

Wenn das Verpackungssystem ein Band oder mehrere Bänder aufweist, kann/können diese(s) in einem optionalen Unterschritt von Schritt (i) zunächst an der Einlage angebracht werden, bspw. indem es/sie durch hierfür in der Einlage vorgesehene Öffnungen geführt wird. Dann können ggf. mittels Band direkt an der Einlage zu befestigende Komponenten des Schlauchsets (bspw. ein Oxygenator) mit dem Band befestigt werden. Hierzu kann die Komponente (bspw. ein Oxygenator) in die dafür vorgesehene Vertiefung bzw. den dafür vorgesehenen Aufnahmeabschnitt der Einlage eingelegt werden und dann mit dem Band befestigt werden.

Weiterhin können in einem Unterschritt von Schritt (i) die Vertiefungen (bzw. Aufnahmeabschnitte) der Einlage mit (den dafür vorgesehenen) Komponenten des Schlauchsets bestückt werden. So kann bspw. ein Schlauch in einer entsprechenden Vertiefung der Einlage eingerollt werden. Schlauchenden können dabei um anderen Komponenten, wie den Oxygenator, herumgeführt werden. Offene Schlauchenden können in eine (abdeckbare) Vertiefung eingelegt werden. Zur Fixierung und - insbesondere bei offenen Schlauchenden - zum weiteren Schutz können Schotts verwendet werden, wie oben beschrieben. So können bspw. offene Schlauchenden, die besonders geschützt werden sollen, zunächst in Schotts eingelegt werden, die in die Einlage (in dafür vorgesehenen Aussparungen) eingesetzt werden, so dass mittels Schotts und einer Abdeckung ein besonders geschützter Raum für die offenen Schlauchenden entsteht. Weitere Komponenten, wie bspw. ein Pumpenkopf, können nach Bedarf auf der Einlage ausgerichtet werden, bspw. mit einem Außengehäuse im Wesentlichen parallel zu einer Kante der Einlage. Bei Bedarf können Komponenten des Schlauchsets auf verschiedene Weise (an der Einlage) befestigt werden, bspw. mittels eines Fixierclips, mittels eines Bands oder mittels eines Schotts, wie oben beschrieben.

Nach der Bestückung der Vertiefungen bzw. Aufnahmeabschnitte der Einlage können diese - soweit vorgesehen - mit einer entsprechenden Abdeckung abgedeckt werden. Die Abdeckungen können bspw. direkt an der Einlage befestigt werden. Es können auch mehrere Abdeckungen (die ihrerseits bspw. bestückt sind, wie oben beschrieben) übereinander angeordnet werden. Hierbei können Abdeckungen, die oberhalb einer anderen Abdeckung angeordnet sind, direkt an der Einlage oder an der unteren Abdeckung befestigt werden. Ggf. können dann weitere Komponenten auf den Abdeckungen angeordnet werden. Eine Befestigung der Abdeckung(en) kann auch (bspw. nach Bestückung) mittels Band erfolgen, so dass die Abdeckung zunächst nur aufgelegt wird.

In Schritt (ii) wird die (vollständig) bestückte Einlage in die Wanne 1 eingebracht und in Schritt (iii) dort reversibel befestigt. Die Befestigung der (bestückten) Einlage in der Wanne kann wie oben beschrieben, bspw. mit dem oben beschriebenen Befestigungsmittel, erfolgen. So kann der Benutzer bspw. das (an der Einlage befindliche) Befestigungsmittel (bzw. dessen oberen Teil) bewegen, so dass eine Verriegelung in der Wanne erzeugt wird, bspw. durch eine Drehbewegung des Befestigungsmittels (z.B. mittels entsprechender Flügel am Befestigungsmittel und korrespondierenden Vorsprüngen in der Wanne).

Insbesondere kann die Wanne nach dem Befestigen der Einlage in der Wanne versiegelt werden (vorzugsweise mit einer Folie, wie oben beschrieben). Die (versiegelte) Wanne kann in einer Sekundärverpackung (bspw. einem Staubschutzbeutel und/oder einem Umkarton) verpackt werden.

In einem weiteren Aspekt stellt die vorliegende Erfindung gemäß Anspruch 19 ein Befestigungsmittel zur reversiblen Befestigung einer Einlage in einer Wanne bereit, wobei das Befestigungsmittel einen Griff und eine Basis umfasst, wobei der Griff vorzugsweise einen Greifabschnitt und einen Fortsatz zur Verbindung mit der Basis aufweist, der durch eine Öffnung in der Einlage hindurchgeführt werden kann. Insbesondere ist im Griff ein Haken (zur Befestigung an einer Schiene bzw. an einem Stab) integriert, der aus dem Griff heraus gelöst werden kann. Wie oben beschrieben entsprechen bevorzugte Ausführungsformen des Befestigungsmittels des erfindungsgemäßen Verpackungssystem bevorzugten Ausführungsformen des erfindungsgemäßen Befestigungsmittels.

Die Erfindung wird im Folgenden anhand der in den Figuren dargestellten weiteren bevorzugten Ausführungsformen näher erläutert. Die Figuren und die nachfolgende Beschreibung sollen die Erfindung in beispielhafter Weise illustrieren. Dabei zeigen:
**Fig. 1** eine schematische Darstellung (Explosivdarstellung) einer bevorzugten Ausführungsform des erfindungsgemäßen Verpackungssystems.
**Fig. 2** eine schematische Darstellung des in Fig. 1 gezeigten Verpackungssystems mit medizinischem Schlauchset, wobei die Wanne und die Einlage mit dem darauf verpackten Schlauchset zur besseren Übersichtlichkeit separat dargestellt sind.
**Fig. 3** eine schematische Darstellung des in Fig. 1 gezeigten Verpackungssystems mit Schlauchset in der Draufsicht, wobei die Einlage mit dem verpackten Schlauchset in der Wanne angeordnet ist.
**Fig. 4** eine schematische Darstellung einer Wanne eines erfindungsgemäßen Verpackungssystems in der Draufsicht.
**Fig. 5** eine schematische Darstellung des vorderen Abschnittes einer Wanne eines erfindungsgemäßen Verpackungssystems in einem Längsschnitt entlang der in Fig. 4 dargestellten Ebene A-A.
**Fig. 6** eine schematische Darstellung der in Fig.4 dargestellten Wanne mit Einlage sowie deren Befestigung mit einem Befestigungsmittel in der Wanne.
**Fig. 7** eine schematische Darstellung der Befestigung einer Einlage in einer Wanne mit einer weiteren Ausführungsform eines Befestigungsmittels (A); eine schematische Darstellung eines Griffs einer weiteren Ausführungsform eines Befestigungsmittels (B); und eine schematische Darstellung eines Griffs einer weiteren Ausführungsform eines Befestigungsmittels (C).
**Fig. 8** eine schematische Darstellung einer weiteren Ausführungsform eines Befestigungsmittels (A); schematische Darstellung des Griffs des in (A) gezeigten Befestigungsmittels (B); sowie schematische Darstellung der Basis des in (A) gezeigten Befestigungsmittels (C).
**Fig. 9 - 12** jeweils schematische Darstellungen verschiedener Ausführungsformen eines Schotts in geschlossenem (A) und geöffnetem (B) Zustand.
**Fig. 13** eine beispielhafte Ausführungsform eines (elastischen) Bandes, das zur Fixierung von Gegenständen (Komponenten des Schlauchsets) in der Verpackung, insbesondere in der Einlage, verwendet werden kann.
**Fig. 14** eine beispielhafte Ausführungsform eines Fixierclips in Perspektivdarstellung (A) und Draufsicht (B), der zur Fixierung von Gegenständen (Komponenten des Schlauchsets) in der Verpackung, insbesondere an der Einlage, verwendet werden kann.
**Fig. 15** eine schematische perspektivische Darstellung einer beispielhaften Abdeckung für eine Vertiefung in der Einlage eines erfindungsgemäßen Verpackungssystems.
**Fig. 16** eine schematische perspektivische Darstellung einer weiteren beispielhaften Abdeckung eines erfindungsgemäßen Verpackungssystems.
**Fig. 17** eine schematische Darstellung einer beispielhaften Einlage eines erfindungsgemäßen Verpackungssystems in der Perspektive (A), in der Draufsicht (B), sowie der in Figur 17B gekennzeichnete Ausschnitt A in Vergrößerung (C).
**Fig. 18** eine schematische Darstellung einer weiteren, beispielhaften Wanne eines erfindungsgemäßen Verpackungssystems in der Draufsicht (A) sowie der in Figur 18A gekennzeichnete Ausschnitt A in Vergrößerung (B).

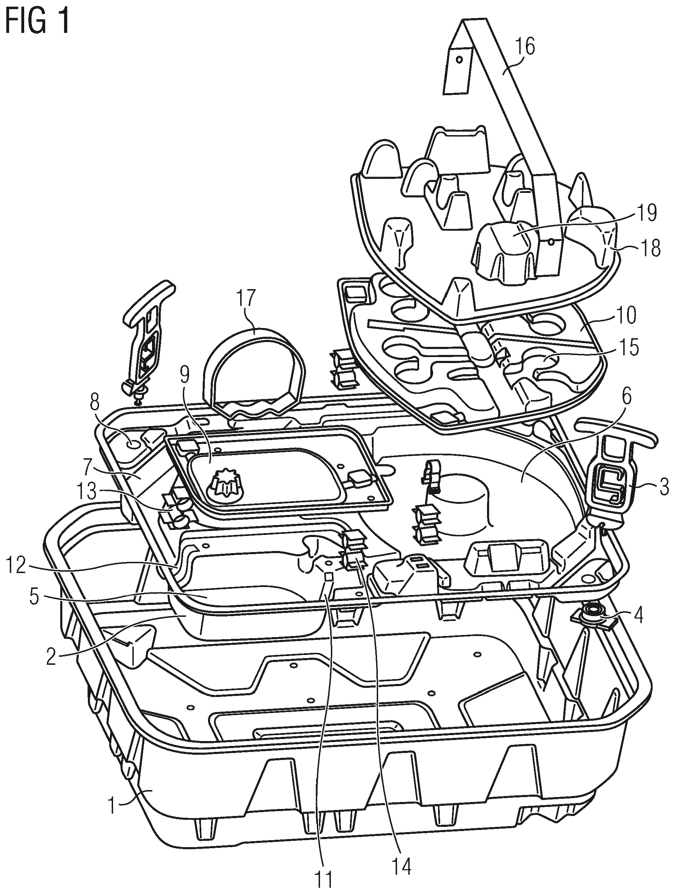

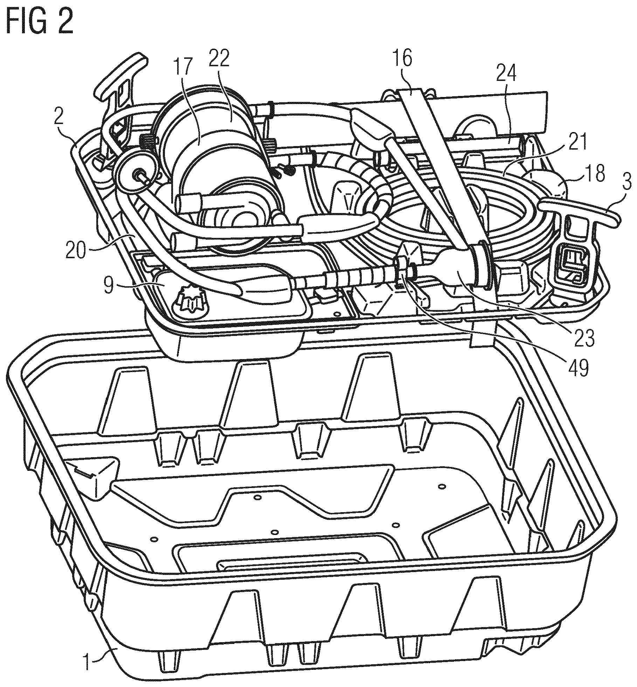

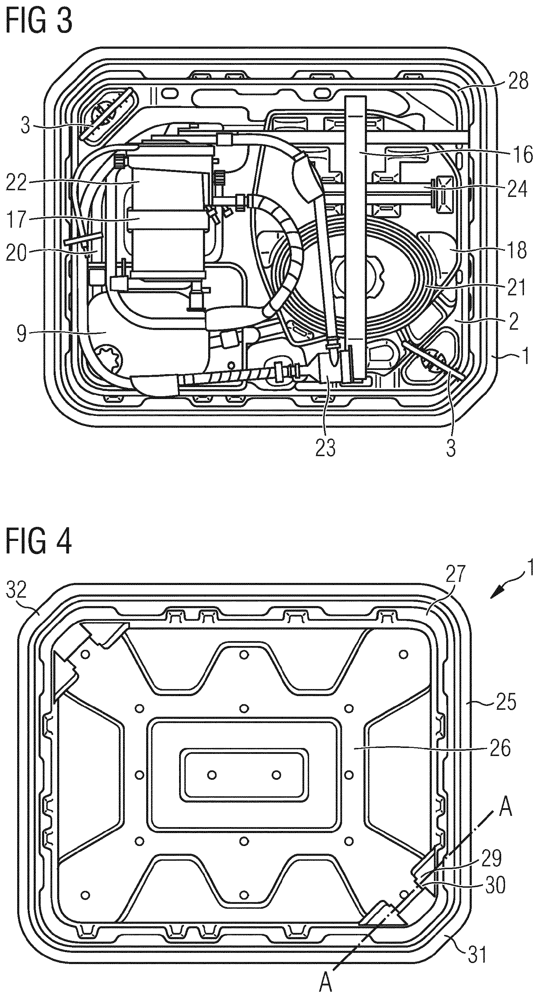

Ein Verpackungssystem für ein medizinisches Schlauchset zur extrakorporalen Therapie mit einer Wanne 1, einer Einlage 2 und einem Befestigungsmittel 3, 4 zur reversiblen Befestigung der Einlage 2 in der Wanne 1 ist in Figuren 1 bis 3 dargestellt. Fig. 1 zeigt das Verpackungssystem ohne Schlauchset; hingegen zeigen die Figuren 2 und 3 jeweils das Verpackungssystem mit einem beispielhaften medizinischen Schlauchset zur extrakorporalen Therapie.

Die Einlage 2 weist Vertiefungen 5, 6, 7 zur Aufnahme eines Schlauches auf. Weiterhin kann die Einlage einen Aufnahmeabschnitt für einen Oxygenator aufweisen (in Figuren 2 und 3 mit Oxygenator dargestellt). Zudem weist die Einlage 2 eine Öffnung 8 aufweisen, durch die das Befestigungsmittel 3, 4 geführt wird, um das Befestigungsmittel 3, 4 vorzugsweise drehbar an der Einlage 2 zu fixieren. Dabei kann die Einlage vorteilhaft in den diagonal gegenüber liegenden Ecken 50, 51 der Einlage 2 jeweils eine Öffnung 8 aufweisen, so dass jeweils ein Befestigungsmittel 3, 4 in den diagonal gegenüber liegenden Ecken 50, 51 der Einlage 2 angeordnet werden kann. Eine Befestigung in (genau) zwei diagonal gegenüber liegenden Ecken 50, 51 der Einlage 2 sorgt für eine sichere Fixierung der Einlage 2 in der Wanne 1. Die Einlage 2 kann weiterhin Öffnungen für ein Band 16, 17 aufweisen. Zudem kann die Einlage 2 Öffnungen zur Befestigung eines Fixierclips 49 aufweisen.

Neben der Wanne 1, der Einlage 2 und dem Befestigungsmittel 3, 4 kann das Verpackungssystem eine oder mehrere Abdeckungen 9, 10 für Vertiefungen 5, 6 in der Einlage 2 aufweisen. Eine oder mehrere Vertiefungen 5, 6 in der Einlage 2 können (in abgedecktem Zustand) seitlich nicht vollständig abgeschlossen sein, sondern zumindest an einer Seite der Vertiefung 5, 6 eine seitliche Aussparung 11, 12 aufweisen, so dass ein oder mehrere Schläuche seitlich in die Vertiefung 5, 6 hinein- oder herausgeführt werden können. Auf diese Weise können zwei oder mehr Vertiefungen 5, 6, 7 miteinander verbunden sein, so dass ein Schlauch durch mehrere Vertiefungen 5, 6 geführt werden kann. Mit anderen Worten, verschiedene, miteinander verbundene Vertiefungen 5, 6 können der Aufnahme desselben Schlauchs dienen. Weiterhin kann das Verpackungssystem ein oder mehrere Schotts 13, 14 umfassen, die einerseits die seitliche Aussparung 11, 12 der Vertiefung 5 formschlüssig ausfüllen können und die ihrerseits Öffnungen für eine möglichst passgenaue Durchführung von Schläuchen aufweisen können. Auf diese Weise kann mittels solcher Schotts 13, 14 in einer Vertiefung 5 der Einlage 2 mit der Abdeckung 9 ein möglichst abgeschlossener Innenraum gebildet werden, der vor Kontaminationen besonders geschützt ist und in den dennoch mittels der Öffnungen der Schotts 13, 14 ein oder mehrere Schläuche (im Wesentlichen kraftschlüssig) hinein- oder hinausgeführt werden können. Darüber hinaus können die Schotts 13, 14 der Arretierung der Schläuche im Verpackungssystem dienen. Ferner kann das Verpackungssystem eine Abdeckung 10 für eine Vertiefung 6 in der Einlage 2 aufweisen, die ihrerseits Vertiefungen 15 aufweist, die bspw. der Aufnahme von (weiteren) Elementen des Schlauchsystems (bspw. weiterer Schläuche) oder der Aufnahme zusätzlicher Artikel, wie Schlauchklemmen, dienen.

Um das Schlauchset in der Einlage 2 zu fixieren, kann das Verpackungssystem ein oder mehrere (bspw. zwei) Bänder 16, 17 umfassen, das/die vorzugsweise elastisch (bspw. aus Silikon) ausgebildet ist/sind. Mehrere Bänder 16, 17 können bspw. sich bspw. hinsichtlich Länge, Breite, Form und/oder Material unterscheiden oder dieselbe Länge, Breite, Form und/oder dasselbe Material aufweisen. Beispielsweise können sich mehrere Bänder 16, 17 lediglich in der Farbgebung unterscheiden (und ansonsten baugleich sein). Das Band bzw. die (elastischen) Bänder 16, 17 können auf einer Seite (zug)fest mit bzw. in der Einlage 2 verankert sein. Auf der anderen Seite können die (elastischen) Bänder 16, 17 derart an der Einlage 2 befestigt sein, dass sie leicht gelöst und bei Bedarf wieder befestigt werden können. Um ein horizontales Verrutschen eines solchen Bandes (bspw. während des Transports oder der Lagerung) zu verhindern oder zu minimieren, kann in einer Abdeckung 18 eine Führung 19 für das Band 16 vorgesehen sein.

Das im Verpackungssystem steril verpackte beispielhafte Schlauchset umfasst einen Schlauch zur Blutführung 20, einen Schlauch zur Gasführung 21, einen Oxygenator 22, sowie einen Pumpenkopf 23. Weiterhin können verschiedene zusätzliche Artikel, die in der Regel zusammen mit dem medizinischen Schlauchset verwendet werden, wie bspw. Schlauchklemmen, eine Spritze 24, Kabelbinder etc., ebenfalls im erfindungsgemäßen Verpackungssystem (steril) verpackt werden.

Wie in Fig. 2 dargestellt, kann das Schlauchset vollständig auf der Einlage 2 verpackt werden, insbesondere außerhalb der Wanne 1. Bei einer Verpackung des Schlauchsets auf der Einlage 2 außerhalb der Wanne 1 sind die Elemente des Schlauchsets und potenzielle zusätzliche Artikel leicht zugänglich und können daher auf einfache Weise auf der Einlage 2 verpackt werden. Danach kann die Einlage 2 (mit dem darauf verpackten Schlauchset) in die Wanne 1 eingebracht und mit den Befestigungsmitteln 3, 4 darin reversibel befestigt werden, wie in Fig. 3 gezeigt. Auf diese Weise wird nicht nur eine besonders einfache Verpackung, sondern auch ein besonders einfaches Auspacken ermöglicht, da durch die reversible Befestigung 3, 4 der Einlage 2 in der Wanne 1, die Einlage 2 mit Schlauchsetkomponenten auf einfache Weise der Wanne 1 entnommen werden kann und die Schlauchsetkomponenten außerhalb der Wanne 1 an ihren Bestimmungsort verbracht und ausgepackt werden können. Eine einfache Verpackung sowie ein einfaches Auspacken mit leichter Zugänglichkeit der Komponenten ist insbesondere unter sterilen Bedingungen von Vorteil, wie bei einer Verpackung im Reinraum und/oder beim Auspacken auf einer Intensivstation.

Nach der Verpackung des Schlauchsets im erfindungsgemäßen Verpackungssystem kann dieses (steril) versiegelt werden, bspw. indem auf die Wanne 1 eine Abdeckung, z.B. eine Folie, zur (sterilen) Versiegelung (des Inhalts) aufgebracht wird. Zum Transport kann die versiegelte Wanne 1 in eine sekundäre Verpackung, bspw. in einen Umkarton, verpackt werden.

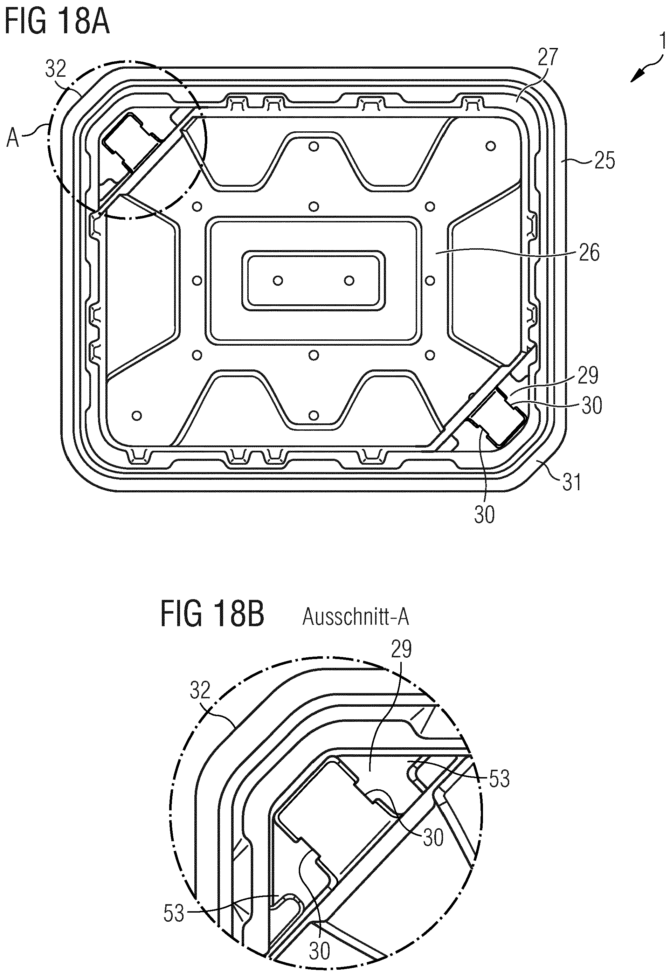

Wie in Fig. 4 und 18 dargestellt, kann die Wanne 1 vorteilhaft einen im Wesentlichen horizontalen oberen Rand 25 aufweisen, so dass die Abdeckung (Folie) auf einfache Weise aufgebracht werden kann. Ein solcher Rand kann bspw. eine Breite von ca. 5 bis 20 mm aufweisen.

Wie in Fig. 4 und 18 beispielhaft dargestellt, kann die Wanne 1 eine im Wesentlichen rechteckige Grundfläche (Boden 26) aufweisen, wobei die "Ecken" des Rechtecks abgerundet oder abgeschrägt sein können. Die Wände der Wanne 1 sind im Wesentlichen senkrecht zum Boden 26 der Wanne 1 ausgebildet. Wie in den Fig. 1 - 4 beispielhaft gezeigt, kann die Wand der Wanne 1 eine Abstufung aufweisen, so dass die Grundfläche (am Boden 26) der Wanne 1 kleiner ist als die durch den oberen Rand 25 gebildeten (Innen-)Fläche. Solche Wannen können leicht gestapelt und daher platzsparend gelagert werden.

Die in Fig. 4 dargestellte Wanne weist in der unteren Hälfte der Wandhöhe (bspw. auf ca. einem Drittel der Wandhöhe), eine im Wesentlichen horizontale Auflagefläche 27 für die Einlage 2 auf. Diese Auflagefläche 27 ist in Fig. 4 im Wesentlichen umlaufend ausgebildet. Die Auflagefläche 27 kann bspw. durch eine Abstufung in der (Innen-)wand erreicht werden. Beim Verpacken des Schlauchsets wird die Einlage 2 auf die Auflagefläche 27 der Innenwand der Wanne 1 aufgelegt, so dass die Unterseite des Außenrands 28 der Einlage 2 zumindest teilweise mit der Auflagefläche 27 der Wanne 1 in Kontakt ist. Die Wanne 1 ist dabei vorteilhaft so dimensioniert, dass die Einlage 2 gleichzeitig auf Auflageflächen 27 an allen Innenwandseiten (bspw. an allen vier Innenwandseiten bei rechteckiger Grundform in Fig. 4) aufliegen kann und die Innenwände der Wanne 1 somit eine horizontale Bewegung der Einlage limitieren bzw. im Wesentlichen verhindern.

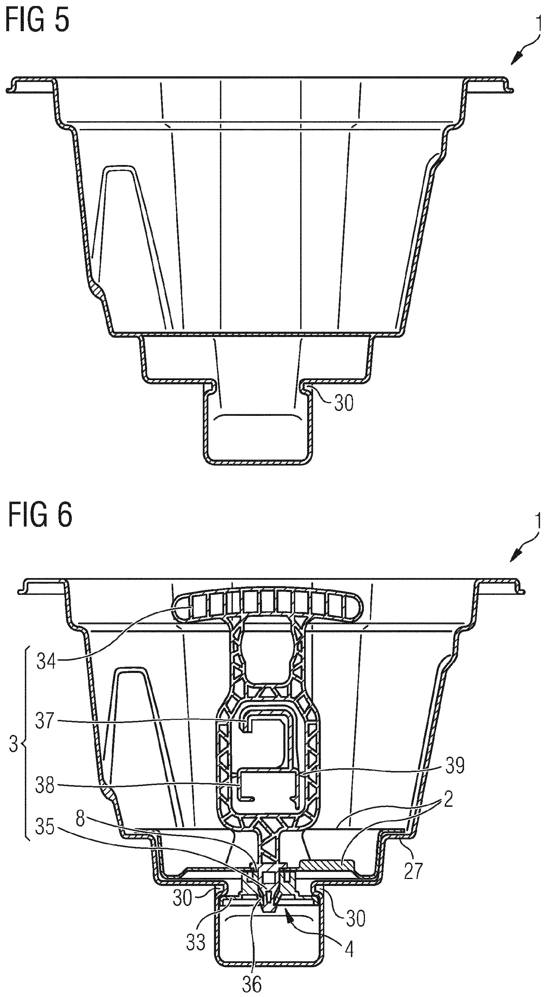

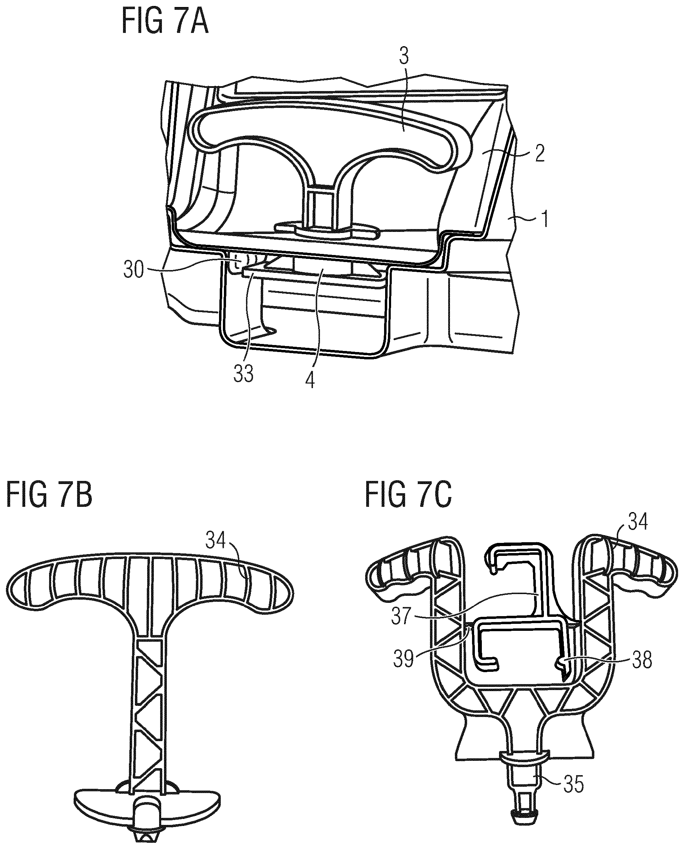

Wie in Fig. 4 und 18 beispielhaft dargestellt, kann die Wanne 1 im bodennahen Bereich vorteilhaft eine Aufnahme 29 für eine Verriegelung durch das Befestigungselement (Befestigungsmittel) 3, 4 aufweisen. Diese Aufnahme 29 wird insbesondere durch einen entsprechend dimensionierten Vorsprung 30 in der (Innen-)Wand der Wanne 1 gebildet, wie beispielhaft in den Figuren 5 und 6 im Längsschnitt (durch eine Ecke der Wanne) gezeigt. Wie in Fig. 4 und 18 beispielhaft gezeigt, können bei rechteckiger Grundform der Wanne 1 zwei Aufnahmen 29 mit (bspw. jeweils zwei) Vorsprüngen 30 in den diagonal gegenüber liegenden Ecken 31, 32 der Wanne 1 angeordnet sein. Dabei können genau zwei Aufnahmen 29 zur Befestigung der Einlage 2 in der Wanne 1 vorgesehen sein. Bei der in den Fig. 4, 5 und 6 gezeigten beispielhaften Wanne 1 weist die Aufnahme 29 zur Befestigung der Einlage 2 in der Wanne 1 zwei, insbesondere gegenüberliegende, Vorsprünge 30 auf, der/die so dimensioniert ist/sind, dass die Flügel 33 einer im Wesentlichen unterhalb der Einlage 2 angeordneten Basis 4 des Befestigungsmittels 3, 4 bei Bewegung (bspw. Drehung) des Befestigungsmittels 3, 4 zumindest teilweise unter die Vorsprünge 30 greifen. Dies ist in den Figuren 6 und 7a beispielhaft dargestellt. Bei entgegengesetzter Bewegung des Befestigungsmittels 3, 4 (bspw. Drehung in Gegenrichtung) greifen die Flügel 33 der Basis 4 des Befestigungsmittels 3, 4 nicht mehr unter die Vorsprünge 30. Das Zusammenwirken der Vorsprünge 30 der (Innen-)wand der Wanne mit den Flügeln 33 der Basis 4 des Befestigungsmittels 3, 4 ermöglicht daher eine Verriegelung durch Bewegung der Flügel 33 relativ zu den Vorsprüngen 30. Bei Drehung des Befestigungsmittels 3, 4 zur reversiblen Befestigung der Einlage 2 in der Wanne 1 kann bspw. eine Drehung um etwa 90° erfolgen, um die Einlage in der Wanne zu befestigen oder zu lösen.

Fig. 6 und 7a zeigt, dass das Befestigungsmittel 3, 4 zur Befestigung der Einlage 2 in der Wanne 1 in der Regel mit der Einlage 2 verbunden ist, wobei das Befestigungsmittel in der Einlage 2 beweglich, bspw. drehbar, sein kann. Die Verbindung des Befestigungsmittels 3, 4 mit der Einlage 2 kann reversibel oder irreversibel ausgeführt sein.

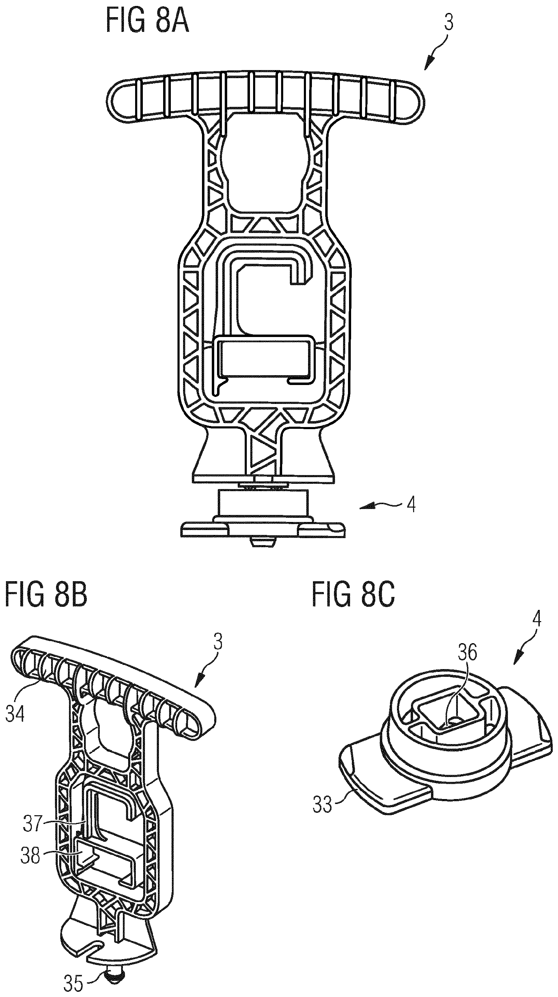

Das Befestigungsmittel weist erfindungsgemäß einen Griff 3 und eine Basis 4 auf, wie in den Figuren 1, 6, 7a, und 8a beispielhaft dargestellt. Dabei kann die Basis 4 des Befestigungsmittels im Wesentlichen unterhalb der Einlage 2 (oder zusätzlich auch innerhalb der Einlage 2) angeordnet sein. Die Basis 4 des Befestigungsmittels kann bspw. der Verriegelung dienen, etwa im Zusammenwirken mit den Vorsprüngen 30 der Wannenwand, wie oben beschrieben. Zu diesem Zweck kann die Basis 4 des Befestigungsmittels einen (bzw. zwei) Flügel 33 aufweisen, wie in Fig. 8c beispielhaft dargestellt. Ein solcher Flügel 33 kann bei Drehung des Befestigungsmittels unter den Vorsprüngen 30 der (Innen-)Wand der Wanne 1 positioniert werden. Dadurch kann eine vertikale Bewegung der Einlage 2 aus der Wanne 1 heraus im Wesentlichen verhindert werden.

Der Griff 3 des Befestigungsmittels kann im Wesentlichen oberhalb (oder zusätzlich auch in) der Einlage 2 angeordnet sein. Der Griff 3 des Befestigungsmittels kann bspw. der Bedienung des Befestigungsmittels durch den Benutzer dienen, bspw. um die Einlage 2 in der Wanne 1 (z.B. durch manuelle Betätigung des Befestigungsmittels) zu befestigen oder zu lösen.

Ein kleiner Teilabschnitt der Basis 4 des Befestigungsmittels kann auch oberhalb der Einlage 2 angeordnet sein und/oder ein kleiner Teilabschnitt des Griffs 3 des Befestigungsmittels kann auch unterhalb der Einlage 2 angeordnet sein, bspw. um beide Teile 3, 4 des Befestigungsmittels miteinander zu verbinden, wie bspw. in den Figuren 6, 7c, und 8b beispielhaft dargestellt. Hier weist der Griff 3 des Befestigungsmittels 3, 4 neben dem Greifabschnitt 34 einen Fortsatz 35 auf, der sich (wenn das Befestigungsmittel 3, 4 in der Einlage 2 befestigt ist) durch die Öffnung und unterhalb der Einlage 2 erstreckt, um den Griff 3 mit der Basis 4 zu verbinden. Mit anderen Worten, wird der Fortsatz 35 des Griffs 3 des Befestigungsmittels durch eine Öffnung 8 der Einlage 2 hindurchgeführt (oder in eine solche Öffnung hinein), um den Griff 3 des Befestigungsmittels auf der Oberseite der Einlage 2 mit Basis 4 des Befestigungsmittels auf der Unterseite der Einlage 2 zu verbinden, wie in Figur 6 beispielhaft dargestellt. Hierzu weist die Basis 4 des Befestigungsmittels vorteilhaft eine Aufnahme 36 für den Fortsatz 35 des Griffs 3 des Befestigungsmittels auf, wie beispielhaft in Fig. 6 und 8C gezeigt. Durch Einbringen des Fortsatzes 35 in die Aufnahme 36 wird eine geeignete Verbindung des Griffs 3 und der Basis 4 des Befestigungsmittels erzielt. Eine solche Verbindung ermöglicht es in der Regel, dass Bewegungen am Griff 3 des Befestigungsmittels, insbesondere an dessen Greifabschnitt 34 ausgeführte Bewegungen - wie bspw. eine Drehbewegung, auf die Basis 4 des Befestigungsmittels übertragen werden. Wenn z.B. am Greifabschnitt 34 durch den Benutzer eine Drehbewegung ausgeführt wird, wird damit auch die (unterhalb der Einlage 2 befindliche) Basis 4 des Befestigungsmittels gedreht. Auf diese Weise kann der Benutzer bspw. durch Drehung des Greifabschnitts 34 die Flügel 33 der Basis 4 des Befestigungsmittels drehen, so dass diese unter den Vorsprüngen 30 der (Innen-)wand positioniert werden (und damit die Einlage 2 in der Wanne 1 befestigt wird). Ebenso kann eine (weitere oder entgegengesetzte) Drehbewegung am Greifabschnitt 34 die Befestigung der Einlage 2 in der Wanne 1 lösen, wenn die Flügel 33 der Basis 4 des Befestigungsmittels nicht mehr unter den Vorsprüngen 30 der (Innen-)wand positioniert sind. Eine derartige Verriegelung kann so ausgeführt werden, dass sie in einer möglichst optimalen Position (bspw. bei größtmöglicher Überlappung der Flügel 33 und Vorsprünge 30; z.B. nach Drehung um ca. 90°) einrastet. Wie in Fig. 8c gezeigt, können die äußeren Seitenränder der Flügel 33 eine abgerundete oder abgeschrägte Form aufweisen.

Die Figuren 6 - 8 zeigen verschiedene beispielhafte Formen des Greifabschnitts 34, wobei diese beispielhaften Greifabschnitte ausreichend breit sind, dass sie gut mit einer Hand bedient werden können (bspw. mindestens 3, 4 oder 5 cm). Der in Fig. 6 dargestellte Griff 3 mit Greifabschnitt 34 ist vorteilhaft so dimensioniert, dass der Greifabschnitt 34 in einem oberen Abschnitt der Wanne 1 angeordnet ist, nämlich kurz unterhalb des Wannenrands 25 (so dass die Oberseite des Griffs 3 des Befestigungsmittels den oberen Wannenrand 25 gerade so nicht überragt). So kann der Greifabschnitt 34 leicht erreicht werden und ist einfach bedienbar. Fig. 7a zeigt einen alternativen Greifabschnitt 34, der in einem mittleren bzw. unteren Abschnitt der Wanne 1 vorgesehen ist.

Wie in den Figuren 6, 7c und 8 beispielhaft gezeigt, kann das Befestigungsmittel 3, 4, insbesondere dessen Griff 3, einen Haken 37 enthalten, der bei Bedarf aus dem Befestigungsmittel 3, 4 heraus gelöst (bspw. herausgebrochen) werden kann. Der Haken 37 kann eine Halterung 38 zur Befestigung des Hakens 37 bspw. an einem Stab oder an einer Schiene aufweisen, wie ebenfalls in den Figuren 6, 7c und 8 beispielhaft gezeigt. In den in Figuren 6 und 7c gezeigten Ausführungsformen ist der Haken 37 lediglich über wenige (bspw. zwei oder drei) möglichst dünne Verbindungsstege 39 im Befestigungsmittel 3, 4 (bzw. in dessen Griff 3) integriert. So werden beim Herauslösen des Hakens 37 aus dem Befestigungsmittel 3, 4 weder das Befestigungsmittel 3, 4 noch der Haken 37 beschädigt. Wie in den Figuren 6, 7 und 8b gezeigt, kann der Griff 3 des Befestigungsmittels 3, 4 mit dem integrierten Haken 37 vorteilhaft einstückig ausgeführt sein, so dass der Griff 3 mit dem integrierten Haken 37 im selben Arbeitsgang hergestellt werden kann. Bei einstückiger Ausführung besteht der Haken 37 in der Regel aus demselben Material wie der Griff 3.

Bei der Befestigung der Einlage 2 in der Wanne 1 mit dem Befestigungsmittel 3, 4 wird zwischen Wanne 1 und Einlage 2 ein Hohlraum ausgebildet. Dieser Hohlraum dient einem sicheren Transport empfindlicher Gegenstände (bspw. des Oxygenators 22 und/oder des Pumpenkopfes 23), da durch den Hohlraum zwischen Einlage 2 und Wanne 1 Vibrationen abgefedert werden können, um das Schlauchset zu schützen.

Die Anordnung des Schlauchsets auf der Einlage 2 kann vorteilhaft in einer Weise erfolgen, die ein Auspacken im Wesentlichen in der Reihenfolge der Verwendung (bspw. in der Reihenfolge des Aufbaus bzw. der Bereitstellung des Schlauchsets für die extrakorporale Therapie) ermöglicht. Auf diese Weise können Fehler bei der Verwendung des Schlauchsets vermieden werden (Poka-Yoke-Prinzip). Hierfür können solche Komponenten des Schlauchsets, die zuerst verwendet werden, möglichst zuoberst verpackt werden, während Komponenten des Schlauchsets, die später (bzw. zuletzt) verwendet werden, weiter unten (bzw. zuunterst verpackt) werden können.

In einigen Ausführungsformen kann die Einlage 2 auch vorteilhaft aufgehängt werden - bspw. mittels eines (elastischen) Bandes 16, 17 des Verpackungssystems. Hierfür kann das (elastische) Band 16, 17 auf einer Seite (zug)fest in der Einlage 2 verankert sein (so können bspw. auch Gegenstände wie der Oxygenator 22 mittels Band 16, 17 an der Einlage 2 befestigt sein). Insbesondere wenn die Einlage 2 aufgehängt wird, kann bei Bedarf zumindest ein Teil des (blutführenden) Schlauches 20 während der Verwendung (bspw. während der Vorbereitung und/oder Durchführung der extrakorporalen Therapie) in der Einlage 2 verbleiben, insbesondere in den für den (blutführenden) Schlauch 20 ausgebildeten Vertiefungen 5, 6, 7 der Einlage 2.

Die für Schläuche vorgesehenen Vertiefungen 5, 6, 7 in der Einlage 2 (bzw. in einer oder mehrerer Abdeckungen 9, 10, 18) können vorteilhaft größer als der Schlauchumfang ausgelegt sein. Die Vertiefungen 5, 6, 7 in der Einlage 2 (bzw. in einer oder mehrerer Abdeckungen 9, 10, 18) können bspw. so ausgelegt sein, dass mehrere Lagen des Schlauchs bzw. mehrere Schläuche darin aufgenommen werden können. Vorteilhaft sind bspw. im Wesentlichen runde oder elliptische Vertiefungen bzw. Aufnahmen für Schläuche, wobei in etwa zentral eine Erhebung angeordnet sein kann, um den Schlauch darum zu wickeln. Dies ist in Fig. 1 sowohl für die Vertiefung 6 in der Einlage 2 dargestellt, als auch für die Abdeckung 18, auf deren Erhebung die Führung 19 für Band 16 angeordnet ist.

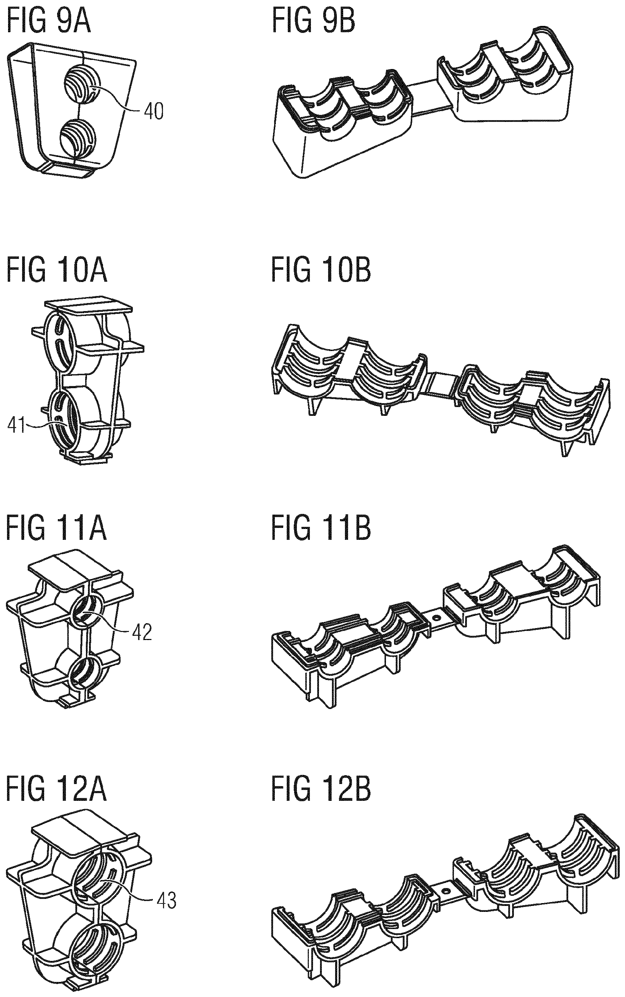

Eine oder mehrere Vertiefungen 5, 6 in der Einlage 2 können dafür vorgesehen sein, mittels einer Abdeckung 9, 10 und geeigneten Mitteln zur Abdichtung 13, 14 besonders geschützte Kompartimente im Verpackungssystem auszubilden. Um einen Schlauch in ein solches besonders geschütztes Kompartiment hinein- oder herauszuführen, können in seitlichen Aussparungen 11, 12 der Vertiefung 5, 6 ein oder mehrere Schotts 13, 14 angeordnet sein (vgl. Fig. 1). Die Schotts 13, 14 können einerseits die seitliche Aussparung 11, 12 der Vertiefung 5, 6 formschlüssig ausfüllen und weisen andererseits ihrerseits Öffnungen 40, 41, 42, 43 für eine möglichst passgenaue Durchführung von Schläuchen auf. Beispielhafte Schotts sind in den Figuren 9 bis 12 gezeigt. Dabei wird in der Regel genau ein Schlauch 20, 21 durch genau eine Öffnung 40, 41, 42, 43 eines Schotts 13, 14 geführt. Die Öffnung 40, 41, 42, 43 des Schotts 13, 14 ist genau an den Schlauchumfang (Schlauchgröße) angepasst, um Kontaminationen des Innenraums der Vertiefung zu vermeiden. So kann ein Verpackungssystem ein oder mehrere Schotts 13, 14, die alle Öffnungen 40, 41, 42, 43 für dieselbe Schlauchgröße bspw. eines (blutführende) Schlauch 20 aufweisen. Alternativ kann ein Verpackungssystem auch ein oder mehrere Schotts 13, 14 enthalten, die Öffnungen 40, 41, 42, 43 für unterschiedliche Schlauchgrößen aufweisen. In den Figuren 9 bis 12 sind vorteilhafte Schotts mit Öffnungen 40, 41, 42, 43 für jeweils zwei Schläuche dargestellt. Jedoch sind auch Schotts 13, 14 für weniger (d.h. nur einen Schlauch) oder mehr Schläuche (bspw. für drei oder vier Schläuche) denkbar, die bspw. in ähnlicher Weise realisiert werden können. Wenn ein Schott 13, 14 Öffnungen 40, 41, 42, 43 für mehrere Schläuche aufweist, können alle Öffnungen 40, 41, 42, 43 des Schotts für dieselbe oder für verschiedene Schlauchgrößen (Schlauchumfänge) ausgelegt sein.

Fig. 9 - 12b zeigen beispielhaft, dass das Schott 13, 14 vorteilhaft entlang der (durch die) Öffnungen 40, 41, 42, 43 aufgeklappt werden kann, bspw. in zwei (Längs-)Hälften des Schotts 13, 14, um die Einbringung der Schläuche in das Schott 13, 14 zu erleichtern. So können die Schläuche einfach in die offenen Schotts 13, 14 eingelegt werden und die Schotts 13, 14 dann verschlossen werden. Das geschlossene Schott 13, 14 mit dem darin eingelegten Schlauch bzw. den darin eingelegten Schläuchen kann dann (formschlüssig) in die Aussparungen 11, 12 der Einlage 2 eingesetzt werden, z.B. durch Einklicken des Schotts 13, 14 in die Einlage 2.

Durch die Schotts 13, 14 kann (bspw. im Zusammenwirken mit einer Abdeckung 9) ein möglichst abgeschlossener Innenraum gebildet werden, der vor Kontaminationen besonders geschützt ist und in den dennoch mittels Öffnungen 40, 41, 42, 43 der Schotts 13, 14 ein oder mehrere Schläuche (auf im Wesentlichen kraftschlüssige Weise) hinein- oder hinausgeführt werden können.

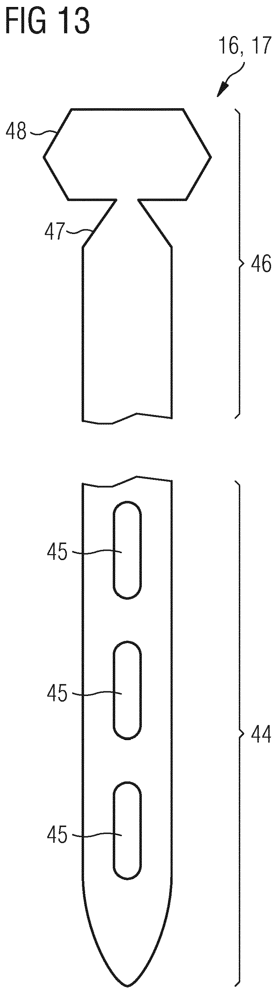

Fig. 13 zeigt eine beispielhafte Ausführungsform des Bandes 16, 17, das zur Fixierung von Gegenständen (Komponenten des Schlauchsets) bspw. in der Einlage 2, wie oben beschrieben, verwendet werden kann. Eine solche Fixierung von Komponenten des Schlauchsets mit einem Band 16, 17 ist in den Fig. 2 und 3 beispielhaft dargestellt. Zur einfachen Befestigung (ohne weitere Zusatzbauteile) und zur schnellen Entriegelung kann das Band 16, 17 in einem Endbereich 44 Öffnungen 45 aufweisen und im anderen Endbereich 46 einen Bereich (beidseitig) verringerter Breite 47, der zum (Band-)Ende hin von einem verbreiterten Bereich 48 abgeschlossen wird.

Bei einer derartigen Formgebung des Bandes 16, 17 kann zum Verschließen (Befestigen) der Endbereich 46 mit dem verbreiterten Bereich 48 durch eine der Öffnungen 45 im anderen Endbereich 44 hindurchgeführt werden, bspw. bis eine weitere Durchführung nicht möglich oder erschwert wird, da sich das Band nach dem Bereich (beidseitig) verringerter Breite 47 wieder verbreitert. Danach verhindert der verbreiterte Bereich 48 an dem einen Ende des Bandes ein unbeabsichtigtes Öffnen des Bandes 16, 17 (Herausrutschen des Bandendes aus der Öffnung, durch die es hindurchgeführt wurde). Zum leichteren Hindurchführen des verbreiterten Bereichs 48 können dessen Seitenränder eine abgerundete oder abgeschrägte Form aufweisen. So kann der verbreiterte Bereich 48 bspw. eine im Wesentlichen sechseckige Form aufweisen, wie in Fig. 13 dargestellt.

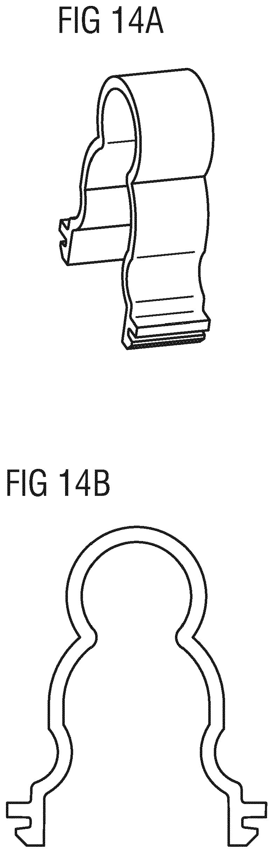

Zusätzlich oder alternativ zu einer Fixierung mit einem Band 16, 17, kann auch ein Fixierclip 49 zur Fixierung von Gegenständen (Komponenten des Schlauchsets) in der Verpackung verwendet werden. Ein solcher Fixierclip 49 ist beispielhaft in Fig. 14 dargestellt. Bspw. kann der Fixierclip 49 vorteilhaft den Pumpenkopf 23 auf der Einlage 2 fixieren, wie in Fig. 2 gezeigt. Auf diese Weise verhindert der Fixierclip 49, dass die die Pumpe 23 sich beim Transport bewegt und dadurch beschädigt werden könnte oder andere Gegenstände bzw. die Verpackung beschädigen könnte. Durch Zusammendrücken des Clips 49 kann der Clip 49 in der Einlage 2 befestigt und anschließend wieder entfernt werden. Der Clip 49 kann vorteilhaft für unterschiedliche Schlauchdurchmesser (bspw. für zwei unterschiedliche Schlauchdurchmesser, wie in Fig. 14 gezeigt) angelegt sein.

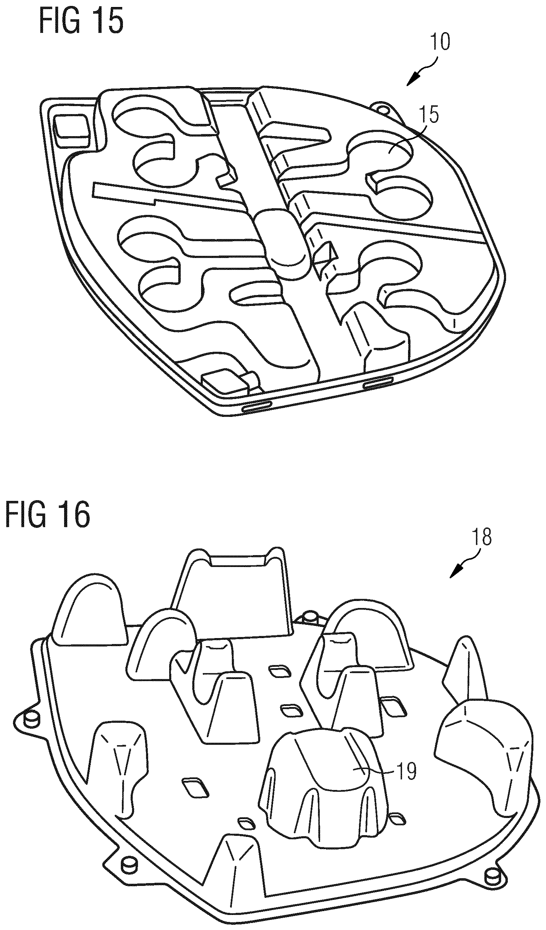

Fig. 15 zeigt eine beispielhafte Abdeckung 10 für eine Vertiefung 6 in der Einlage 2. Die Abdeckung 10 kann zur Abdeckung eines in einer Vertiefung 6 der Einlage 2 (bspw. eines darin gelagerten Schlauchs 20) dienen. Zudem kann die Abdeckung 10 ihrerseits eine oder mehrere Vertiefung(en) zur im Wesentlichen formschlüssigen Aufnahme bzw. Anordnung von Komponenten des Schlauchsets oder von zusätzlichen Gegenständen, die im Zusammenhang mit dem medizinischen Schlauchset verwendet werden können, aufweisen. So weist die in Fig. 15 gezeigte beispielhafte Abdeckung Vertiefungen 15 zur Aufnahme von Schlauchklemmen auf.

Fig. 16 zeigt eine weitere beispielhafte Abdeckung 18, die wie in Figur 1 gezeigt, als Abdeckung für die Abdeckung 10 ausgelegt sein kann. Auf diese Weise können mehrere Abdeckungen platzsparend übereinander (gestapelt) angeordnet werden, wobei vorteilhaft Komponenten des Schlauchsets bzw. zusätzliche Gegenstände voneinander getrennt auf übersichtliche Weise verpackt werden können. Dabei kann die zuoberst angeordnete Abdeckung 18 eine Führung 19 für das Band 16 aufweisen. So kann mit Hilfe des Bands 16 die Abdeckung 18 - sowie ggf. darunter befindliche Abdeckung(en) - fixiert werden. Auch die Abdeckung 18 kann ihrerseits eine oder mehrere Vertiefung(en) bzw. Erhebungen zur im Wesentlichen formschlüssigen Aufnahme bzw. Anordnung von Komponenten des Schlauchsets oder von zusätzlichen Gegenständen, die im Zusammenhang mit dem medizinischen Schlauchset verwendet werden können, aufweisen. So weist die in Fig. 16 gezeigte beispielhafte Abdeckung Erhebungen zur Aufnahme/Anordnung einer Spritze 24 auf. Zudem kann die Führung 19 auf einer im Wesentlichen kreisrunden oder elliptischen Erhebung angeordnet sein, die ihrerseits die Aufnahme bzw. Anordnung eines (aufgerollten) Schlauchs 21 (um die Erhebung herum) ermöglicht. Dabei sind die Vertiefungen oder Erhebungen zur Aufnahme von Komponenten des Schlauchsets oder von zusätzlichen Gegenständen vorteilhaft derart angeordnet, dass darin angeordnete Gegenstände ebenfalls durch das in der Führung 19 geführte Band zusätzlich fixiert werden können.

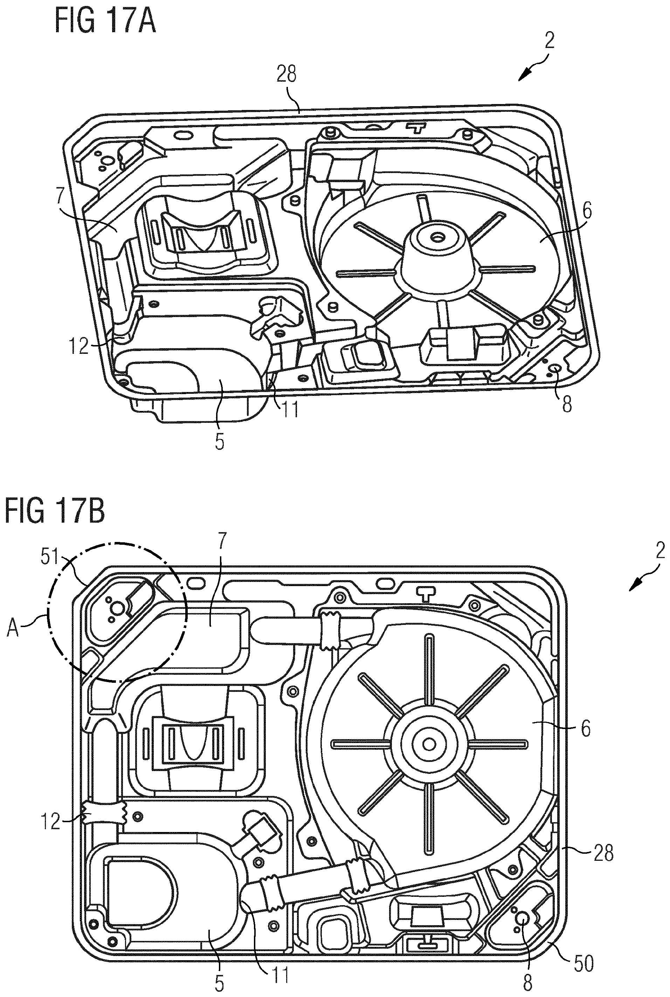

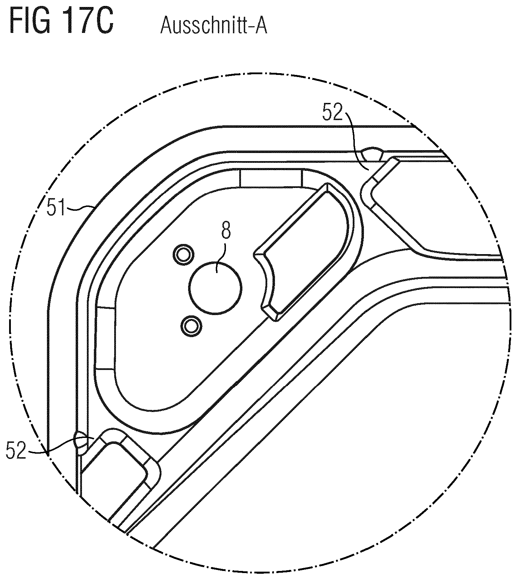

Eine weitere beispielhafte Einlage 2 ist in Fig. 17 perspektivisch (A) und in einer Draufsicht (B) dargestellt. Der Ausschnitt A aus Fig. 17B ist in Fig. 17C vergrößert dargestellt. Auch diese beispielhafte Einlage 2 weist Vertiefungen 5, 6, 7 zur Aufnahme eines Schlauches auf. Die Vertiefungen 5, 6 in der Einlage 2 können (in abgedecktem Zustand) seitlich nicht vollständig abgeschlossen sein, sondern zumindest an einer Seite der Vertiefung 5, 6 eine seitliche Aussparung 11, 12 aufweisen, so dass ein oder mehrere Schläuche seitlich in die Vertiefung 5, 6 hinein- oder herausgeführt werden können. Weiterhin kann die Einlage einen Aufnahmeabschnitt für einen Oxygenator aufweisen (in Figuren 2 und 3 mit Oxygenator dargestellt). Die Einlage 2 kann weiterhin Öffnungen für ein Band 16, 17 aufweisen. Zudem kann die Einlage 2 Öffnungen zur Befestigung eines Fixierclips 49 aufweisen. Vorteilhaft weist die Einlage 2 eine Öffnung 8 auf, durch die das Befestigungsmittel 3, 4 geführt wird, um das Befestigungsmittel 3, 4 vorzugsweise drehbar an der Einlage 2 zu fixieren, wie oben beschrieben. Dabei kann die Einlage vorteilhaft in den diagonal gegenüber liegenden Ecken 50, 51 der Einlage 2 jeweils eine Öffnung 8 aufweisen, so dass jeweils ein Befestigungsmittel 3, 4 in den diagonal gegenüber liegenden Ecken 50, 51 der Einlage 2 angeordnet werden kann.

Weiterhin kann eine Ecke 51 der Einlage 2 vorteilhaft eine pfeilförmige Vertiefung 52 (oder eine pfeilförmige Erhebung) aufweisen, wie in Fig. 17C dargestellt, um die Stabilität der Einlage 2 in der Wanne 1 weiter zu erhöhen. Zu diesem Zweck kann die Wanne 1, wie in Fig. 18 dargestellt, in der korrespondierenden Ecke 32 eine im Wesentlichen formschlüssig korrespondierende pfeilförmige Vertiefung 53 (oder eine pfeilförmige Erhebung) aufweisen. Dabei zeigt der Kopf des Pfeils der pfeilförmigen Vertiefung 52, 53 (oder Erhebung) der Einlage 2 bzw. der Wanne 1 in diagonaler Richtung zur Ecke 51, 32 (Rand) der Einlage 2 bzw. Wanne 1. Die Spitze des Pfeilkopfs kann abgerundet oder abgeflacht (d.h. ohne eigentliche "Spitze") ausgeführt werden, wie in den Figuren 17C und 18B ersichtlich. Das hintere, breite Ende des Pfeilkopfes ist im Wesentlichen senkrecht zur diagonalen Ausrichtung des Pfeils angeordnet. Die beiden Ränder der Vertiefung (oder Erhebung), die das hintere, breite Ende des Pfeilkopfes darstellen, sind daher im Wesentlichen senkrecht zur diagonalen Ausrichtung des Pfeils angeordnet. So kann die Einlage 2 noch besser in der Wanne 1 fixiert werden. Zu diesem Zweck ist es besonders vorteilhaft, wenn die Einlage 2 und die Wanne 1 jeweils zwei (formschlüssig zwischen Einlage 2 und Wanne 1 korrespondierende) pfeilförmige Vertiefungen 52, 53 (oder Erhebungen), insbesondere in diagonal gegenüber liegenden Ecken 50, 51, 31, 32 der Einlage 2 bzw. Wanne 1 aufweisen. Dabei kann es sich um dieselben diagonal gegenüber liegenden Ecken 50, 51 der Einlage 2 handeln, die vorteilhaft eine Öffnung 8 für das Befestigungsmittel 3, 4 aufweisen. Alternativ kann es sich auch um diagonal gegenüber liegende Ecken handeln, die keine Öffnungen 8 aufweisen. Besonders vorteilhaft ist es, wenn alle vier Ecken der Einlage 2 bzw. Wanne 1 jeweils (formschlüssig zwischen Einlage 2 und Wanne 1 korrespondierende) pfeilförmige Vertiefungen 52, 53 (oder Erhebungen) aufweisen. Auf diese Weise wird selbst bei einem Sturz oder Fall (bspw. während des Transports) verhindert, dass die Einlage 2 in der Wanne 1 verrutscht.

Ein beispielhaftes Schlauchset kann auf einfache Weise in dem erfindungsgemäßen Verpackungssystem verpackt werden. Dabei wird zunächst das medizinische Schlauchset auf der Einlage 2 außerhalb der Wanne 1 verpackt; dann die Einlage 2 mit dem verpackten Schlauchset in die Wanne 1 eingebracht; und dann wird die Einlage 2 (mit dem verpackten Schlauchset) reversibel in der Wanne 1 befestigt.

Zur Illustration dieses Verpackungsverfahrens wird im Folgenden die Verpackung eines beispielhaften Schlauchsets umfassend einen Schlauch zur Blutführung 20, einen Schlauch zur Gasführung 21, einen Oxygenator 22, sowie einen Pumpenkopf 23 (und ggf. verschiedene zusätzliche Artikel, die in der Regel zusammen mit dem medizinischen Schlauchset verwendet werden, wie bspw. Schlauchklemmen, eine Spritze 24, Kabelbinder etc.) in dem in den Figuren 1 bis 18 dargestellten beispielhaften Verpackungssystem näher erläutert. Dabei wird zunächst das Befestigungsmittel 3,4 an der Einlage 2 befestigt. Hierfür kann die Basis 4 des Befestigungsmittels so (unterhalb der Einlage 2 bzw. der darin befindlichen Öffnung 8) in Position gebracht werden, dass der Griff 3 des Befestigungsmittels durch die Einlage 2 (nämlich durch die Öffnung 8) hindurch mit der Basis 4 verbunden werden kann (bspw. durch eine Einrast-Funktion, z.B. des Fortsatzes 35 in der Aufnahme 36). Im beispielhaft gezeigten Verpackungssystem kann dies zweimal, nämlich an diagonal gegenüberliegenden Ecken, durchgeführt werden, um zwei Befestigungsmittel an der Einlage 2 anzubringen. Ein Band 17 wird durch hierfür in der Einlage 2 vorgesehene Öffnungen eingefädelt. Die Abdeckungen 10 und 18 werden mit den jeweils hierfür vorgesehenen Komponenten (bspw. zusätzliche Artikel, die in der Regel zusammen mit dem medizinischen Schlauchset verwendet werden, wie bspw. Schlauchklemmen, eine Spritze 24, Kabelbinder etc.) bestückt. Die Reihenfolge der vorgenannten Schritte ist beliebig, da die Einlage 2 sowie die zu bestückenden Abdeckungen 10 und 18 jeweils unabhängig voneinander behandelt werden können. Dann kann ein Oxygenator 22 in die (hierfür vorgesehene Vertiefung bzw. den hierfür vorgesehenen Aufnahmeabschnitt der) Einlage 2 eingelegt werden. Dies kann vorteilhaft so geschehen, dass im beispielhaften Verpackungssystem der Bluteinlass- bzw. Auslassport des Oxygenators 22 nach rechts bzw. die Gaslinie "short" des Oxygenators 22 nach links zeigt (vgl. Fig. 2 und 3). Der Oxygenator 22 kann dann mit dem Band 17 an der Einlage 2 arretiert werden. Danach können die Schlauchenden des Schlauchsets (bspw. im Uhrzeigersinn) um den Oxygenator 22 herumgeführt werden und der Schlauch in der Vertiefung 6 der Einlage 2 eingerollt werden. Die offenen Schlauchenden können aufeinander (bspw. mit (der jeweiligen) Schlauchklemme) in der Vertiefung 5 der Einlage 2 positioniert werden. Die Schläuche können dann mit einem Schott 13, 14 (bspw. mit zwei oder drei Schotts 13, 14) in den entsprechenden Aussparungen 11, 12 der Vertiefung 5 arretiert werden. Die Vertiefung 6 (mit dem eingerollten Schlauch) kann dann mit der Abdeckung 10 abgedeckt werden, wobei die Abdeckung 10 an der Einlage 2 arretiert werden kann. Der Pumpenkopf 23 kann nach Bedarf auf der Einlage 2 ausgerichtet werden, bspw. mit dem Außengehäuse im Wesentlichen parallel zur Kante von Einlage 2. Mittels eines Fixierclips 49 kann der Schlauch oberhalb an der Bluteinlassseite des Pumpenkopfs 23 auf der Einlage 2, bspw. direkt am weißen Spiralschlauch, arretiert werden. Die Abdeckung 18 kann an der Einlage 2 (oder an der Abdeckung 10) befestigt (eingeklemmt) werden, bspw. so dass sich die Abdeckung 18 direkt auf der Abdeckung 10 befindet. Dann kann bspw. ein Füllset (priming linie) in Einlage 2, bspw. auf Abdeckung 18, eingelegt werden. Dabei können im beispielhaften Verpackungssystem die offenen Schlauchenden vorteilhaft nach links (zum Oxygenator 22) zeigen. Die Schlauchenden können (bspw. gegen den Uhrzeigersinn) um den Oxygenator 22 herumgeführt werden. Die offenen Schlauchenden (Coulder-Kupplungen) können (bspw. mit (den jeweiligen) Schlauchklemmen) übereinander der Vertiefung 5 der Einlage 2 eingelegt werden. Die Schläuche können dann mit einem Schott 13, 14 bspw. übereinander in der entsprechenden Aussparung 11, 12 der Vertiefung 5 arretiert werden. Weiterhin können in der Vertiefung 5 optional weitere Komponenten (bspw. zusätzliche Artikel, die in der Regel zusammen mit dem medizinischen Schlauchset verwendet werden, wie bspw. ein Tannenbaumkonnektor und Luer-Kappen etc.) eingelegt werden. Danach kann die Abdeckung 9 auf der Einlage 2 arretiert werden. Auf der Abdeckung 18 kann ein weiterer Schlauch, bspw. eine (grüne) Gaslinie ("long") eingerollt und abgelegt werden. Das Band 16 kann an der Einlage 2 durch entsprechende Öffnungen eingefädelt werden. Die Abdeckung 18 und darin befindliche Komponenten (bspw. eine (grüne) Gaslinie ("long"), eine Spritze 24, ein Füllset (priming linie), etc.) werden so mittels Band 17 an der Einlage 2 fixiert. Die vollständig bestückte Einlage 2 wird dann in die Wanne 1 eingebracht. Die Einlage 2 wird reversibel an der Wanne 1 befestigt, bspw. indem der Griff 3 (bzw. die Griffe 3) gedreht werden, z.B. im Uhrzeigersinn um ca. 90°.

Nach dem Befestigen der Einlage 2 in der Wanne 1 kann diese versiegelt werden, vorzugsweise luftdicht über die gesamte Öffnung der Wanne 1 hinweg. Hierzu kann z.B. eine Folie (Topseal) verwendet werden. Die (versiegelte) Wanne 1 kann in einer Sekundärverpackung (bspw. einem Umkarton) verpackt werden. Hierbei kann die Wanne 1 zunächst in einen Staubschutzbeutel eingelegt werden und dann ggf. in einen Karton verpackt werden.

### Bezugszeichenliste

- 1: Wanne
- 2: Einlage
- 3: oberer Teil des Befestigungsmittel (Griff)
- 4: unterer Teil des Befestigungsmittel (Basis)
- 5, 6, 7: Vertiefung in der Einlage zur Aufnahme eines Schlauches
- 8: Öffnung
- 9, 10: Abdeckung für Vertiefung
- 11, 12: seitliche Aussparung der Vertiefung 5
- 13, 14: Schott
- 15: Vertiefung in Abdeckung 10 zur Aufnahme einer Schlauchklemme
- 16, 17: Band
- 18: Abdeckung für (Vertiefungen einer) weiteren Abdeckung
- 19: Führung für Band 16
- 20: Schlauch zur Blutführung
- 21: Schlauch zur Gasführung
- 22: Oxygenator
- 23: Pumpenkopf
- 24: Spritze
- 25: oberer Rand der Wanne
- 26: Boden der Wanne
- 27: Auflagefläche
- 28: Außenrand der Einlage
- 29: Aufnahme für eine Verriegelung durch das Befestigungselement (Befestigungsmittel)
- 30: Vorsprung in (Innen-)Wand der Wanne
- 31, 32: diagonal gegenüberliegende Ecken der Wanne
- 33: Flügel im unteren Teil (bspw. der Basis 4) des Befestigungsmittels
- 34: Greifabschnitt des (oberen Teils des) Befestigungsmittels
- 35: Fortsatz zur Verbindung mit dem unteren Teil (Basis) des Befestigungsmittels
- 36: Aufnahme für Fortsatz
- 37: Haken
- 38: Halterung für Haken
- 39: Verbindungssteg zur Integration des Hakens im Befestigungsmittel
- 40, 41, 42, 43: Öffnung in Schott
- 44: Endbereich des Bandes mit Öffnung(en)
- 45: Öffnungen im Endbereich 44 des Bandes
- 46: Endbereich 46 des Bandes mit einen Bereich (beidseitig) verringerter Breite und verbreitertem Bereich
- 47: Bereich (beidseitig) verringerter Breite (im Endbereich 46 des Bandes)
- 48: verbreiterter Bereich (im Endbereich 46 des Bandes)
- 49: Fixierclip
- 50, 51: diagonal gegenüberliegende Ecken der Einlage
- 52: pfeilförmige Vertiefung in Ecke der Einlage
- 53: pfeilförmige Vertiefung in Ecke der Wanne

## Patentansprüche

1. Verpackungssystem für ein medizinisches Schlauchset zur extrakorporalen Therapie umfassend:
- eine Wanne (1), die zur Aufnahme einer Einlage (2) sowie zur reversiblen Befestigung der Einlage 2 in der Wanne 1 ausgebildet ist,
- eine Einlage (2) mit einer oder mehreren Vertiefungen (5, 6, 7) zur Aufnahme von Komponenten des Schlauchsets,
wobei zwischen Wanne (1) und Einlage (2) ein Hohlraum ausgebildet ist, **dadurch gekennzeichnet, dass**
das Verpackungssystem weiterhin
- ein Befestigungsmittel (3, 4) zur reversiblen Befestigung der Einlage (2) in der Wanne (1)
umfasst,
wobei das Befestigungsmittel (3, 4) einen Griff (3) und eine Basis (4) aufweist, wobei der Griff (3) einen Greifabschnitt (34) und einen Fortsatz (35) zur Verbindung mit der Basis (4) aufweist, der durch eine Öffnung (8) in der Einlage (2) hindurchgeführt ist.

2. Verpackungssystem nach Anspruch 1, **dadurch gekennzeichnet, dass** das Verpackungssystem steril ist.

3. Verpackungssystem nach Anspruch 1 oder 2, **dadurch gekennzeichnet, dass** die Einlage (2) Vertiefungen (5, 6, 7) zur Aufnahme eines oder mehrerer Schläuche (20, 21), sowie vorzugsweise einen Aufnahmeabschnitt für einen Oxygenator (22), aufweist.

4. Verpackungssystem nach einem der vorhergehenden Ansprüche, **dadurch gekennzeichnet, dass** sich die Einlage (2) im Wesentlichen über den gesamten Bereich der Wanne (1) erstreckt,
oder
**dadurch gekennzeichnet, dass** Einlage derart ausgebildet, dass alle Komponenten des Schlauchsets sowie ggf. alle weiteren zu verpackenden Gegenstände vollständig auf der Einlage verpackt werden können.

5. Verpackungssystem nach einem der vorhergehenden Ansprüche, **dadurch gekennzeichnet, dass** die Wanne (1) an ihrer Innenwand eine im Wesentlichen horizontale Auflagefläche (27) aufweist, auf der die Einlage (2) abgelegt werden kann.

6. Verpackungssystem nach Anspruch 7, **dadurch gekennzeichnet, dass** der Griff (3) des Befestigungsmittels (3, 4) mit der Einlage (2) verbunden und derart ausgelegt ist, dass er zur Entnahme und/oder zum Transport der Einlage (2), insbesondere mit den darauf verpackten Komponenten des Schlauchsets, sowie ggf. mit allen weiteren verpackten Gegenständen, dient.

7. Verpackungssystem nach einem der Ansprüche 1 bis 6, **dadurch gekennzeichnet, dass** das Befestigungsmittel (3, 4) drehbar ist, die Basis (4) des Befestigungsmittels (3, 4) einen Flügel (33) aufweist und die Innenwand der Wanne (1) einen oder mehrere Vorsprünge (30) aufweist, die je nach Position des Flügels (33) eine Befestigung oder ein Lösen der Einlage (2) in der Wanne (1) bewirken.

8. Verpackungssystem nach Anspruch 7, **dadurch gekennzeichnet, dass** bei Drehung des Befestigungsmittels (3, 4) der Flügel 33 derart gegenüber dem einen oder mehreren Vorsprüngen (30) der Innenwand der Wanne (1) verschoben wird, dass ein Befestigen oder Lösen der Einlage (2) in der Wanne (1) bewirkt wird.

9. Verpackungssystem nach einem der vorhergehenden Ansprüche, wobei das Verpackungssystem ferner eine Abdeckung (9, 10, 18) umfasst, die eine Vertiefung (5, 6, 15) oder einen Aufnahmeabschnitt zumindest teilweise abdeckt.

10. Verpackungssystem nach Anspruch 9, wobei die Abdeckung (9) eine Vertiefung (5) der Einlage (2) abdeckt und wobei die Vertiefung (5) in der Einlage (2) seitlich nicht vollständig abgeschlossen ist, sondern zumindest an einer Seite der Vertiefung (5) eine Aussparung (11, 12) aufweist, so dass ein oder mehrere Schläuche (20, 21) seitlich in die Vertiefung (5) hinein- oder herausgeführt werden können.

11. Verpackungssystem nach Anspruch 9 oder 10, **dadurch gekennzeichnet, dass** das Verpackungssystem ferner ein Schott (13, 14) zur formschlüssigen Anordnung in der seitlichen Aussparung (11, 12) der Vertiefung (5) umfasst, durch das ein Schlauch (20, 21) hindurchgeführt werden kann.

12. Verpackungssystem nach einem der Ansprüche 9 - 11, wobei das Verpackungssystem eine Abdeckung (10) für eine Vertiefung (6) der Einlage (2) umfasst, die Vertiefungen (15) zur Aufnahme von Gegenständen zur Verwendung mit dem medizinischen Schlauchset, bspw. Schlauchklemmen, aufweist.

13. Verpackungssystem nach einem der vorhergehenden Ansprüche, wobei das Verpackungssystem ferner ein Band (16, 17) zur Fixierung von einer oder mehreren Komponenten des Schlauchsets umfasst,
wobei das Verpackungssystem bevorzugt ferner eine Abdeckung (18) (für eine Vertiefung (6) der Einlage (2) oder zur Abdeckung einer weiteren Abdeckung (10) umfasst, die eine Führung (19) für das Band (16) aufweist.

14. Verpackungssystem nach einem der vorhergehenden Ansprüche, wobei das Verpackungssystem ferner einen Fixierclip umfasst.

15. Verpackungssystem nach einem der vorhergehenden Ansprüche, **dadurch gekennzeichnet, dass** das Verpackungssystem ein Auspacken im Wesentlichen in der Reihenfolge der Verwendung der verpackten Gegenstände ermöglicht.

16. Verwendung des Verpackungssystems nach einem der Ansprüche 1 - 15 zum Verpacken eines medizinischen Schlauchsets.

17. Verfahren zum Verpacken eines medizinischen Schlauchsets in einem Verpackungssystems nach einem der Ansprüche 1 - 15, umfassend die folgenden Schritte:
(i) Verpacken des medizinischen Schlauchsets auf der Einlage (2) außerhalb der Wanne (1);
(ii) Einbringen der Einlage (2) mit dem verpackten Schlauchset in die Wanne (1); und
(iii) reversible Befestigung der Einlage (2) in der Wanne (1) mittels eines Befestigungsmittels (3, 4).

18. Verfahren nach Anspruch 17, wobei die Wanne (1) nach dem Befestigen der Einlage (2) in der Wanne (1) versiegelt wird.

19. Befestigungsmittel (3, 4) eines Verpackungssystems nach einem der Ansprüche 1-15 zur reversiblen Befestigung der Einlage (2) in der Wanne (1), umfassend einen Griff (3) und eine Basis (4), wobei der Griff (3) einen Greifabschnitt (34) und einen Fortsatz (35) zur Verbindung mit der Basis (4) aufweist, der durch eine Öffnung (8) in der Einlage (2) hindurchgeführt werden kann.

20. Befestigungsmittel (3, 4) nach Anspruch 19, wobei im Griff (3) ein Haken (37) integriert ist, der bei Bedarf aus dem Griff (3) herausgelöst werden kann.

## Claims

1. A packaging system for a medical tubing set for extracorporeal therapy, comprising:
- a tray (1), which is configured to receive an inlay 2 and to reversibly fasten the inlay (2) in the tray (1), and
- an inlay (2) with one or more recesses (5, 6, 7) for accommodating components of the tubing set,
wherein a cavity is formed between the tray (1) and the inlay (2),
**characterized in that**
the packaging system further comprises
- a fastening means (3, 4) for reversibly fastening the inlay (2) in the tray (1), wherein the fastening means (3, 4) comprises a handle (3) and a base (4), the handle (3) having a gripping section (34) and an extension (35) for connection to the base (4), which is passed through an opening (8) in the inlay (2).

2. The packaging system according to claim 1, **characterized in that** the packaging system is sterile.

3. The packaging system according to claim 1 or 2, **characterized in that** the inlay (2) has recesses (5, 6, 7) for receiving one or more tubes (20, 21), and preferably a receiving section for an oxygenator (22).

4. The packaging system according to any one of the preceding claims, **characterized in that** the inlay (2) extends essentially over the entire area of the tray (1), or
**characterized in that** the inlay (1) is configured, such that all components of the tubing set and all potentially further items to be packaged may be fully packaged on the inlay.

5. The packaging system according to any one of the preceding claims, **characterized in that** the tray (1) has a substantially horizontal support surface (27) on its inner wall, on which the inlay (2) can be placed.

6. The packaging system according to any one of claims 1 to 5, **characterized in that** the handle (3) of the fastening means (3, 4) is connected to the inlay (2) and is adapted so as to serve for removal and/or transport of the inlay (2), in particular with components of the tubing set packaged thereon and all potential further packaged items.

7. The packaging system according to any one of claims 1 to 6, **characterized in that** the fastening means (3, 4) is rotatable, the base (4) of the fastening means (3, 4) has a wing (33) and the inner wall of the tray (1) has one or more projections (30) which, depending on the position of the wing (33), cause the inlay (2) to be fastened or detached in the tray (1).

8. The packaging system according to claim 7, **characterized in that** the wing (33) is displaced relative to the one or more projections (30) of the inner wall of the tray (1) in such a way that fastening or detaching of the inlay (2) in the tray (1) is achieved, when the fastening means (3, 4) is rotated.

9. The packaging system according to any one of the preceding claims, wherein the packaging system further comprises a cover (9, 10, 18), which at least partially covers a recess (5, 6, 15) or a receiving section.

10. The packaging system according to claim 9, wherein the cover (9) covers a recess (5) of the inlay (2) and wherein the recess (5) in the inlay (2) is not completely closed off laterally, but has a cut-out (11, 12) at least on one side of the recess (5), such that one or more tubes (20, 21) can be laterally inserted into or extend laterally out of the recess (5).

11. The packaging system according to claim 9 or 10, **characterized in that** the packaging system further comprises a partition (13, 14) for form-fitting arrangement in the lateral cut-out (11, 12) of the recess (5), through which a tube (20, 21) can be passed.

12. The packaging system according to any one of claims 9 - 11, wherein the packaging system comprises a cover (10) for a recess (6) of the inlay (2), which has recesses (15) for receiving objects for use with the medical tubing set, e.g. tube clamps.

13. The packaging system according to any one of the preceding claims, wherein the packaging system further comprises a strap (16, 17) for fixing one or more components of the tubing set, wherein preferably the packaging system further comprises a cover (18) for a recess (6) of the inlay (2) or for covering another cover (10), which has a guide (19) for the strap (16).

14. The packaging system according to any one of the preceding claims, wherein the packaging system further comprises a fixing clip.

15. The packaging system according to any one of the preceding claims, **characterized in that** the packaging system enables unpacking substantially in the order of use of the packaged articles.

16. Use of a packaging system according to any one of claims 1 - 15 for packaging a medical tubing set.

17. A method of packaging a medical tubing set in a packaging system according to any one of claims 1 - 15, comprising the following steps:
(i) packaging the medical tubing set on the inlay (2) outside the tray (1);
(ii) inserting the inlay (2) with the packaged tubing set into the tray (1); and
(iii) reversible fastening of the inlay (2) in the tray (1) by means of a fastening means (3, 4).

18. The method according to claim 17, wherein the tray (1) is sealed after the fastening of the inlay (2) in the tray (1).

19. A fastening means (3, 4) of a packaging system according to any one of claims 1 - 15 for reversibly fastening the inlay (2) in the tray (1), comprising a handle (3) and a base (4), the handle (3) having a gripping section (34) and an extension (35) for connection to the base (4), which can be passed through an opening (8) in the inlay (2).

20. The fastening means (3, 4) according to claim 19, wherein a hook (37) is integrated in the handle (3), which can be released from the handle (3) if necessary.

## Revendications

1. Système d'emballage pour un ensemble médical de tuyaux flexibles pour la thérapie extracorporelle comportant :
- une cuve (1) qui est conçue pour la réception d'un insert (2) ainsi que pour le montage réversible de l'insert 2 dans la cuve 1,
- un insert (2) avec un ou plusieurs creux (5, 6, 7) pour la réception de composants de l'ensemble de tuyaux flexibles,
dans lequel une cavité est conçue entre la cuve (1) et l'insert (2),
**caractérisé en ce que**
le système d'emballage comporte en outre
- un moyen de montage (3, 4) pour le montage réversible de l'insert (2) dans la cuve (1),
dans lequel le moyen de montage (3, 4) comprend une poignée (3) et une base (4), dans lequel la poignée (3) comprend une partie de préhension (34) et un appendice (35) pour la liaison à la base (4), lequel est guidé à travers une ouverture (8) dans l'insert (2).

2. Système d'emballage selon la revendication 1, **caractérisé en ce que** le système d'emballage est stérile.

3. Système d'emballage selon la revendication 1 ou 2, **caractérisé en ce que** l'insert (2) comprend des creux (5, 6, 7) pour la réception d'un ou de plusieurs tuyaux flexibles (20, 21), ainsi que de préférence une partie de réception pour un oxygénateur (22).

4. Système d'emballage selon l'une des revendications précédentes, **caractérisé en ce que** l'insert (2) s'étend essentiellement sur la totalité de la zone de la cuve (1),
ou
**caractérisé en ce que** l'insert est conçu de telle sorte que tous les composants de l'ensemble de tuyaux flexibles ainsi qu'éventuellement tous les autres objets à emballer puissent être emballés complètement sur l'insert.

5. Système d'emballage selon l'une des revendications précédentes, **caractérisé en ce que** la cuve (1) comprend au niveau de sa paroi interne une surface d'appui essentiellement horizontale (27), sur laquelle l'insert (2) peut être déposé.

6. Système d'emballage selon la revendication 7, **caractérisé en ce que** la poignée (3) du moyen de montage (3, 4) est reliée à l'insert (2) et disposée de telle sorte qu'elle soit utile pour le retrait et/ou pour le transport de l'insert (2), en particulier avec les composants de l'ensemble de tuyaux flexibles emballés sur celui-ci, ainsi qu'éventuellement avec tous les autres objets emballés.

7. Système d'emballage selon l'une des revendications 1 à 6, **caractérisé en ce que** le moyen de montage (3, 4) est rotatif, la base (4) du moyen de montage (3, 4) comprend une ailette (33) et la paroi interne de la cuve (1) comprend une ou plusieurs saillies (30) qui provoquent, en fonction de la position de l'ailette (33), un montage ou une libération de l'insert (2) dans la cuve (1).

8. Système d'emballage selon la revendication 7, **caractérisé en ce que** lors de la rotation du moyen de montage (3, 4) l'ailette (33) est déplacée par rapport aux une ou plusieurs saillies (30) de la paroi interne de la cuve (1) de telle sorte qu'un montage ou une libération de l'insert (2) dans la cuve (1) soit provoqué.

9. Système d'emballage selon l'une des revendications précédentes, le système d'emballage comportant en outre un couvercle (9, 10, 18) qui recouvre au moins en partie un creux (5, 6, 15) ou une partie de réception.

10. Système d'emballage selon la revendication 9, dans lequel le couvercle (9) recouvre un creux (5) de l'insert (2) et dans lequel le creux (5) dans l'insert (2) est fermé latéralement de manière incomplète, mais comprend au moins au niveau d'une face du creux (5) un évidement (11, 12), de sorte qu'un ou plusieurs tuyaux flexibles (20, 21) peuvent être guidés latéralement à l'intérieur ou en dehors du creux (5).

11. Système d'emballage selon la revendication 9 ou 10, **caractérisé en ce que** le système d'emballage comporte en outre une cloison (13, 14) pour l'agencement par complémentarité de forme dans l'évidement (11, 12) latéral du creux (5), à travers laquelle un tuyau flexible (20, 21) peut être guidé.

12. Système d'emballage selon l'une des revendications 9 à 11, le système d'emballage comportant un couvercle (10) pour un creux (6) de l'insert (2), lequel comprend des creux (15) pour la réception d'objets pour l'utilisation avec l'ensemble médical de tuyaux flexibles, par exemple de colliers de serrage pour tuyaux flexibles.

13. Système d'emballage selon l'une des revendications précédentes, le système d'emballage comportant en outre une bande (16, 17) pour la fixation d'un ou de plusieurs composants de l'ensemble de tuyaux flexibles,
le système d'emballage comportant de préférence en outre un couvercle (18) pour un creux (6) de l'insert (2) ou pour le recouvrement d'un autre couvercle (10), lequel comprend un guidage (19) pour la bande (16).

14. Système d'emballage selon l'une des revendications précédentes, le système d'emballage comportant en outre un clip de fixation.

15. Système d'emballage selon l'une des revendications précédentes, **caractérisé en ce que** le système d'emballage permet un déballage essentiellement dans l'ordre de l'utilisation des objets emballés.

16. Utilisation du système d'emballage selon l'une des revendications 1 à 15 pour l'emballage d'un ensemble médical de tuyaux flexibles.

17. Procédé pour l'emballage d'un ensemble médical de tuyaux flexibles dans un système d'emballage selon l'une des revendications 1 à 15, comportant les étapes suivantes :
(i) emballage de l'ensemble médical de tuyaux flexibles sur l'insert (2) à l'extérieur de la cuve (1) ;
(ii) introduction de l'insert (2) avec l'ensemble de tuyaux flexibles emballé dans la cuve (1) ; et
(iii) montage réversible de l'insert (2) dans la cuve (1) au moyen d'un moyen de montage (3, 4).

18. Procédé selon la revendication 17, dans lequel la cuve (1) est scellée après le montage de l'insert (2) dans la cuve (1).

19. Moyen de montage (3, 4) d'un système d'emballage selon l'une des revendications 1 à 15 pour le montage réversible de l'insert (2) dans la cuve (1), comportant une poignée (3) et une base (4), dans lequel la poignée (3) comprend une partie de préhension (34) et un appendice (35) pour la liaison à la base (4), lequel peut être guidé à travers une ouverture (8) dans l'insert (2).

20. Moyen de montage (3, 4) selon la revendication 19, dans lequel dans la poignée (3) est intégré un crochet (37) qui peut être détaché de la poignée (3) si nécessaire.
